# EUROPÄISCHE PATENTSCHRIFT

(11) **EP 1 970 371 B1**
(45) Veröffentlichungstag und Bekanntmachung des Hinweises auf die Patenterteilung: **25.08.2010**
(21) Anmeldenummer: 08004762.4
(22) Anmeldetag: 14.03.2008
(51) Int. Cl.: C07D 215/24, C07D 471/04, C09K 11/06, H01L 51/50, H01L 51/54, H01L 33/00

(54) **Pyrido(3,2-h)chinazoline und/oder deren 5,6-Dihydroderivate, deren Herstellungsverfahren und diese enthaltendes dotiertes organisches Halbleitermaterial**
Pyrido(3,2-h)chinazolins and/or 5,6-Dihydro derivatives thereof, method for their manufacture and endowed organic semiconductor material containing them
Pyrido(3,2-h)chinazoline et/ou leurs dérivés 5,6-dihydro, leur procédé de fabrication et matériau semi-conducteur organique en étant doté

(30) Priorität: 16.03.2007 DE 102007012794
(43) Veröffentlichungstag der Anmeldung: 17.09.2008
(73) Patentinhaber: Novaled AG, 01307 Dresden (DE)
(72) Erfinder: Lux, Andrea, 01277 Dresden (DE); Saalbeck, Josef, 34260 Kaufungen (DE); Kussler, Manfred, 79774 Albbruck (DE)
(74) Vertreter: Bittner, Thomas L.

(56) Entgegenhaltungen:
- EP-A- 1 786 050
- WO-A-2008/022633
- US-A1- 2004 068 115
- BALDO M A ET AL: "Very high-efficiency green organic light-emitting devices based on electrophosphorescence" APPLIED PHYSICS LETTERS, AIP, AMERICAN INSTITUTE OF PHYSICS, MELVILLE, NY, Bd. 75, Nr. 1, 5. Juli 1999 (1999-07-05), Seiten 4-6, XP012023409 ISSN: 0003-6951
- TOMIOKA YUKIHIKO ET AL: "Studies on aromatic nitro compounds. V. A simple one-pot preparation of o-aminoaroylnitriles from some aromatic nitro compounds" CHEMICAL AND PHARMACEUTICAL BULLETIN, PHARMACEUTICAL SOCIETY OF JAPAN, TOKYO, Bd. 33, Nr. 4, 1. Januar 1985 (1985-01-01), Seiten 1360-1366, XP009102906 ISSN: 0009-2363

## Beschreibung

Die vorliegende Erfindung betrifft Pyrido[3,2-h]chinazoline und/oder deren 5,6-Dihydroderivate, Verfahren zu deren Herstellung sowie dotierte organische Halbleitermaterialien, in denen diese Chinazoline eingesetzt werden.

Seit der Demonstration von organischen Leuchtdioden und Solarzellen 1989 [C.W. Tang et al., Appl. Phys. Lett. 51 (12), 913 (1987)] sind aus organischen Dünnschichten aufgebaute Bauelemente Gegenstand intensiver Forschung. Derartige Schichten besitzen vorteilhafte Eigenschaften für die genannten Anwendungen, wie z.B. effiziente Elektrolumineszenz für organische Leuchtdioden, hohe Absorptionskoeffizienten im Bereich des sichtbaren Lichtes für organische Solarzellen, preisgünstige Herstellung der Materialien und Fertigung der Bauelemente für einfachste elektronische Schaltungen, u.a. Kommerzielle Bedeutung hat bereits der Einsatz organischer Leuchtdioden für Displayanwendungen.

Die Leistungsmerkmale (opto-)elektronischer mehrschichtiger Bauelemente werden unter anderem von der Fähigkeit der Schichten zum Transport der Ladungsträger bestimmt. Im Falle von Leuchtdioden hängen die ohmschen Verluste in den Ladungstransportschichten beim Betrieb mit der Leitfähigkeit zusammen, was einerseits direkten Einfluss auf die benötigte Betriebsspannung hat, andererseits aber auch die thermische Belastung des Bauelements bestimmt. Weiterhin kommt es in Abhängigkeit von der Ladungsträgerkonzentration der organischen Schichten zu einer Bandverbiegung in der Nähe eines Metallkontakts, die die Injektion von Ladungsträgern erleichtert und damit den Kontaktwiderstand verringern kann. Ähnliche Überlegungen führen auch für organische Solarzellen zu dem Schluss, dass deren Effizienz auch durch die Transporteigenschaften für Ladungsträger bestimmt wird.

Durch Dotierung von Löchertransportschichten mit einem geeigneten Akzeptormaterial (p-Dotierung) bzw. von Elektronentransportschichten mit einem Donatormaterial (n-Dotierung) kann die Ladungsträgerdichte in organischen Festkörpern (und damit die Leitfähigkeit) beträchtlich erhöht werden. Darüber hinaus sind in Analogie zur Erfahrung mit anorganischen Halbleitern Anwendungen zu erwarten, die gerade auf Verwendung von p- und n-dotierten Schichten in einem Bauelement beruhen und anders nicht denkbar wären. In US 5,093,698 ist die Verwendung von dotierten Ladungsträgertransportschichten (p-Dotierung der Löchertransportschicht durch Beimischung von akzeptorartigen Molekülen, n-Dotierung der Elektronentransportschicht durch Beimischung von donatorartigen Molekülen) in organischen Leuchtdioden beschrieben.

Folgende Ansätze sind bisher für die Verbesserung der Leitfähigkeit von organischen aufgedampften Schichten bekannt:
1. Erhöhung der Ladungsträgerbeweglichkeit durch
   a) Verwendung von Elektronentransportschichten bestehend aus organischen Radikalen (US 5,811,833),
   b) Erzeugung hoch geordneter Schichten, die eine optimale Überlappung der π-Orbitale der Moleküle erlauben,
2. Erhöhung der Dichte der beweglichen Ladungsträger durch
   a) Reinigung und schonende Behandlung der Materialien, um die Ausbildung von Ladungsträgerhaftstellen zu vermeiden,
   b) Dotierung organischer Schichten mittels
      aa) anorganischer Materialien (Alkalimetalle: J. Kido et al., US 6,013,384; J. Kido et al., Appl. Phys. Lett. 73, 2866 (1998), Oxidationsmittel wie Iod, SbCl₅ etc.)
      bb) organischer Materialien (TNCQ: M. Maitrot et al., J. Appl. Phys., 60 (7), 2396-2400 (1986), F4TCNQ: M. Pfeiffer et al., Appl. Phys. Lett., 73 (22), 3202 (1998), BEDT-TTF: A. Nollau et al., J. Appl. Phys., 87 (9), 4340 (2000), Naphthalendicarbonsäureamide: M.Thomson et al., WO03088271, kationische Farbstoffe: A.G. Werner, Appl. Phys. Lett. 82, 4495 (2003))
      cc) Organometallverbindungen (Metallocene: M.Thomson et al., WO03088271)
      dd) Metallkomplexe (Ru⁰(terpy)₃: K. Harada et al., Phys. Rev. Lett. 94, 03G641. (2005)

Während es für die p-Dotierung bereits ausreichend starke, organische Dotanden gibt (F4TCNQ), stehen für dien-Dotierung nur anorganische Materialien, z.B. Cäsium, zur Verftigung. Durch Einsatz dieser konnte auch bereits eine Verbesserung der Leistungsparameter von OLEDs erzielt werden. So erzielt man durch Dotierung der Löchertransportschicht mit dem Akzeptormaterial F4TCNQ eine drastische Reduzierung der Betriebsspannung der Leuchtdiode (X. Zhou et al., Appl. Phys. Lett., 78 (4), 410 (2001).). Ein ähnlicher Erfolg ist durch die Dotierung der elektronen-transportierenden Schicht mit Cs oder Li zu erzielen (J. Kido et al., Appl. Phys. Lett., 73 (20), 2866 (1998); J.-S. Huang et al, Appl. Phys. Lett., 80, 139 (2002)).

Ein großes Problem bei der n-Dotierung war lange Zeit, dass für diese nur anorganische Materialien zur Verfügung standen. Die Verwendung von anorganischen Materialien hat jedoch den Nachteil, dass die verwendeten Atome bzw. Moleküle aufgrund ihrer geringen Größe leicht im Bauelement diffundieren können und somit eine definierte Herstellung z.B. scharfer Übergänge von p- dotierten zu n-dotierten Gebieten erschweren.

Die Diffusion sollte demgegenüber bei Verwendung großer, Raum ausfüllender, organischer Moleküle als Dotanden eine untergeordnete Rolle spielen, da Platzwechselvorgänge nur unter Überwindung höherer Energiebarrieren möglich sind.

Aus der WO 2005/086251 A2 ist die Verwendung eines Metallkomplexes als n-Dotand zur Dotierung eines organischen halbleitenden Matrixmaterials zur Veränderung der elektrischen Eigenschaften desselben bekannt, wobei die Verbindung bezüglich des Matrixmaterials einen n-Dotanden darstellt. Dabei wird vorgeschlagen, als Dotandenverbindung einen neutralen elektronenreichen Metallkomplex mit einem Zentralatom als vorzugsweise neutrales oder geladenes Übergangsmetallatom mit einer Valenzelektronenzahl von zumindest 16 einzusetzen.

Es ist seit vielen Jahren insbesondere bei organischen polymeren Halbleitermaterialien bekannt, daß ein wirksamer Elektronentransfer von einem Dotanden (beispielsweise Natrium) auf die organische Matrix (beispielsweise Polyacetylen) nur möglich ist, wenn die Differenz zwischen HOMO-Energieniveau (= Ionisationspotential) des Dotanden und dem LUMO-Energieniveau (= Elektronenaffinität) der Matrix möglichst gering ist.

Zur Bestimmung des Ionsiationspotentials ist Ultraviolett-Photoelektronenspektroskopie (UPS) die bevorzugte Methode (z.B. R. Schlaf et al., J. Phys. Chem. B 103, 2984 (1999)). Eine verwandte Methode, inverse Photoelektronenspektroskopie (IPES), wird zur Bestimmung von Elektronenaffinitäten herangezogen (zB. W. Gao et. al, Appl. Phys. Lett. 82, 4815 (2003), ist jedoch weniger etabliert. Alternativ können die Festkörperpotentiale durch elektrochemische Messungen von Oxidationspotentialen Eₒₓ bzw. Reduktionspotentialen E_{red} in der Lösung, z.B. durch Cyctovoltammetrie (engl. Cyclic Voltammetry, CV), abgeschätzt werden (zB. J.D. Anderson, J. Amer. Chem. Soc. 120, 9646 (1998)). Mehrere Arbeiten geben empirische Formeln zur Umrechnung der elektrochemischen Spannungsskala (Oxidationspotentiale) in die physikalische (absolute) Energieskala (Ionisationspotentiale) an, z.B. B.W. Andrade et al., Org. Electron. 6, 11 (2005); T.B. Tang, J. Appl. Phys. 59, 5 (1986); V.D. Parker, J. Amer. Chem. Soc. 96, 5656 (1974); L.L. Miller, J. Org. Chem. 37, 916 (1972), Y. Fu et al., J. Amer. Chem. Soc. 127, 7227 (2005). Zwischen Reduktionspotential und Elektronenaffinität ist keine Korrelation bekannt, da sich Elektronenaffinitäten nur schwer messen lassen. Deshalb werden vereinfacht die elektrochemische und die physikalische Energieskala über IP=4.8 eV+ e*Eₒₓ (vs. Ferrocen/Ferrocenium) bzw. EA=4.8eV +e*E_{red} (vs. Ferrocen/Fenrocenium) ineinander umgerechnet, wie in B.W. Andrade, Org. Electron. 6, 11 (2005) (siehe dort auch Ref. 25-28) beschrieben. Die Umrechnung von verschiedenen Standardpotentialen bzw. Redoxpaaren wird beispielsweise in A.J. Bard, L.R. Faulkner, "Electrochemical Methods: Fundamentals and Applications", Wiley, 2. Auflage 2000 beschrieben.

Aus der obigen Darstellung ergibt sich somit, daß die genaue Ermittlung sämtlicher Energiewerte gegenwärtig nicht möglich ist und die dargestellten Werte lediglich als Richtgrößen aufgefaßt werden können.

Beim n-Dotieren fungiert der Dotand als Elektronendonator und überträgt Elektronen auf eine Matrix, welche sich durch eine genügend hohe Elektronenaffinität auszeichnet. Das heißt die Matrix wird reduziert. Durch den Transfer von Elektronen vom n-Dotanden auf die Matrix wird die Ladungsträgerdichte der Schicht erhöht. Inwieweit ein n-Dotand in der Lage ist, Elektronen an eine geeignete, elektronenaffine Matrix abzugeben und dadurch die Ladungsträgerdichte und damit einhergehend die elektrische Leitfähigkeit zu erhöhen, hängt wiederum von der relativen Lage des HOMOs des n-Dotanden und des LUMOs der Matrix relativ zueinander ab. Wenn das HOMO des n-Dotanden über dem LUMO der elektronenaffinen Matrix liegt, kann ein Elektronentransfer stattfinden. Wenn das HOMO des n-Dotanden unter dem LUMO der elektronenaffinen Matrix liegt, kann ebenfalls ein Elektronentransfer stattfinden, vorausgesetzt, dass die Energiedifferenz zwischen den beiden Orbitalen ausreichend niedrig ist, um eine gewisse thermische Population des höheren Energieorbitals zu ermöglichen. Je Meiner diese Energiedifferenz ist, desto höher sollte die Leitfähigkeit der resultierenden Schicht sein. Die höchste Leitfähigkeit ist jedoch zu erwarten für den Fall, dass das HOMO-Niveau des n-Dotanden über dem LUMO-Niveau der elektronenaffinen Matrix liegt. Die Leitfähigkeit ist praktisch messbar und ein Maß dafür, wie gut der Elektronenübertrag vom Donor auf den Akzeptor funktioniert, vorausgesetzt, dass die Ladungsträgermobilitäten verschiedener Matrizen vergleichbar sind.

Die Leitfähigkeit einer Dünnschichtprobe wird mit der 2-Punkt-Methode gemessen. Dabei werden auf ein Substrat Kontakte aus einem leitfähigen Material aufgebracht, z.B. Gold oder Indium-Zinn-Oxid. Danach wird die zu untersuchende Dünnschicht großflächig auf das Substrat aufgebracht, so dass die Kontakte von der Dünnschicht überdeckt werden. Nach Anlegen einer Spannung an die Kontakte wird der dann fließende Strom gemessen. Aus der Geometrie der Kontakte und der Schichtdicke der Probe ergibt sich aus dem so bestimmten Widerstand die Leitfähigkeit des Dünnschichtmaterials. Die 2-Punkt-Methode ist zulässig, wenn der Widerstand der Dünnschicht wesentlich größer ist als der Widerstand der Zuleitungen oder der Kontaktwiderstand. Experimentell wird dies dadurch einen genügend hohen Kontaktabstand gewährleistet, und dadurch kann die Linearität der Strom-Spannungs-Kennlinie überprüft werden.

Untersuchungen der Erfinder haben gezeigt, daß Metallkomplexdotanden der Struktur I 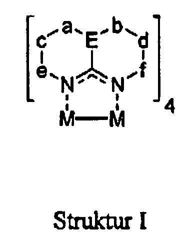 vorteilhaft als Dotanden für ein organisches Matrixmaterial eingesetzt werden können, da ein solcher Dotand das oben beschriebene Diffusionsproblem löst. Aus diesem Grunde wurde ein Dotand mit der Struktur Ia 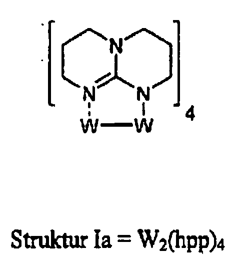 als Dotand für herkömmliche Elektronentransportmaterialien, wie Alq₃ (Tris(8-hydroxyquinolinato)-aluminium) oder BPhen (4,7-Diphenyl-1,10-phenanthrolin), getestet.

Das Gasphasenionisationspotential des Dotanden mit der Struktur 1a beträgt 3,6 eV. Das entsprechende Ionisationspotential des Festkörpers kann nach Y. Fu et al. (J. Am. Chem. Soc. 2005, 127, 7227-7234) abgeschätzt werden und beträgt etwa 2,5 eV.

Die Ergebnisse sind in der folgenden Tabelle 1 dargestellt.

**Tabelle 1: CV-Daten, empirisch ermittelte LUMO-Energien, und gemessene Leitfähigkeiten verschiedener Elektronentransportmaterialien (BAlq₂ = Bis(2-methyl-8-quinolinato)-4-(phenylphenolato)aluminium-(III), BPhen = Bathophenanthrolin, Alq3: (Tris(8-hydroxyquinoline-)aluminium**

| Matrixmaterial | LUMO in cV (ermittelt über CV mit Fc/Fc⁺ als internen Standard) | σ (Leitfähigkeit) in S/cm undotiert | σ. (Leitfähigkeit) in S/cm bei einer Dotierkonzentration von 5 mol% |
|---|---|---|---|
| Alq₃ | 2,4 | < 1E-10 | 9,2E-8 |
| BPhen | 2,42 | < 1E-10 | 4E-9 |
| BAlq₂ | 2,39 | < 1E-10 | 8e-8 |

Wie aus der Tabelle 1 entnommen werden kann, sind die erzielten Leitfähigkeiten mit den bekannten Matrixmaterialien noch unzureichend und sehr gering.

US 2004 068 115 offenbart beispielsweise redoxdotierbare Matrix materialien.

Aufgabe der vorliegenden Erfindung ist es, verbesserte Matrixmaterialien für dotierte organische Halbleitermaterialien bereitzustellen, die die Nachteile des Stands der Technik überwinden. Insbesondere sollen die Leitfähigkeiten unter Einsatz der Matrixmaterialien verbessert werden. Auch soll ein Halbleitermaterial zur Verfügung gestellt werden, das eine erhöhte Ladungsträgerdichte und effektive Ladungsträgerbeweglichkeit sowie eine verbesserte Leitfähigkeit aufweist. Das Halbleitermaterial soll eine hohe thermische Stabilität zeigen, die sich beispielsweise aus höheren Glasübergangspunkten, höheren Sublimationstemperaturen und höheren Zersetzungstemperaturen ergibt.

Schließlich ist es eine Aufgabe der Erfindung, ein Herstellungsverfahren für die Matrixmaterialien bereitzustellen.

Die Aufgabe der Erfindung wird gelöst durch Pyrido[3,2-h]chinazoline der folgenden Struktur 8: 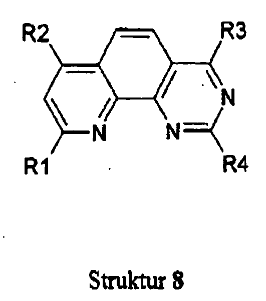 und/oder deren 5,6-Dihydroderivate
wobei:
R¹ und R², substituiert oder unsubstituiert, Aryl, Heteroaryl, Alkyl der Formel CHR₂ mit R=Alkyl, mit C₁-C₂₀ oder Alkyl der Formel CR₃ mit R=Alkyl, mit C₁-C₂₀ ist;
R³ ausgewählt ist aus H, substituiert oder unsubstituert, Alkyl mit C₁-C₂₀, Aryl und Heteroaryl;
R⁴ ausgewählt ist aus H, substituiert oder unsubstituiert, Alkyl mit C₁-C₂₀, Aryl, Heteroaryl, NH₂, NHR mit R=Alkyl mit C₁-C₂₀, NR₂ mit R=Alkyl mit C₁-C₂₀, N-Alkylatyl, N-Aryl₂, Carbazolyl, Dibenzazepinyl und CN;
In den Pyrido[3,2-h]chinazolinen der Struktur 8 sollen R¹ und R² vorzugsweise nicht H, CN, Halogen, NH₂, NO, NO₂, OH, SH, OR, SR, COOR, CHO sowie Alkyl der Formel CH₃ und CH₂R mit R=Alkyl mit C₁-C₂₀ sein.

Diese Pyrido[3,2-h]chinazoline und/oder deren 5,6-Dihydroderivate können als Matrixmaterialien in dotierten organischen Halbleitermaterialien eingesetzt werden und führen dann zu verbesserten Lcitfähigkeitsergebnissen.

Die Pyrido[3,2-h]chinazoline und/oder deren 5,6-Dihydroderivate können gemäß einem Verfahren hergestellt werden, das die folgenden Schritte umfaßt:
(i) Umsetzung eines 2,4-disubstituierten Chinolinons der Struktur 4 mit einem Aldehyd in Gegenwart einer Base zur Darstellung eines Benzylidenhydrochinolinons 5 gemäß dem folgenden Reaktionsschema: 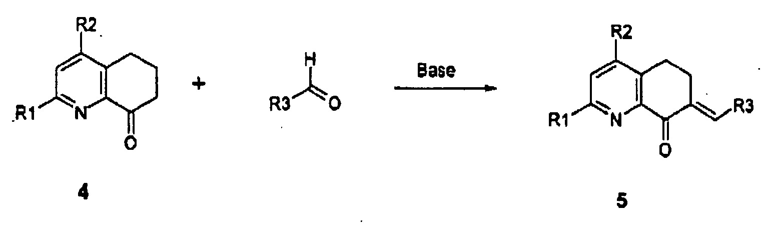
(ii) Umsetzung des Benzylidenhydrochinolinons **5** mit Benzamidiniumhydrochlorid unter basischen Bedingungen zur Darstellung eines 1,4,5,6-Tetrahydro-pyrido[3,2-h]chinazolins **6** und anschließender Oxidation zur Darstellung eines 5,6-Dihydro-pyrido[3,2-h]chinazolins 7 gemäß dem folgenden Reaktionsschema: 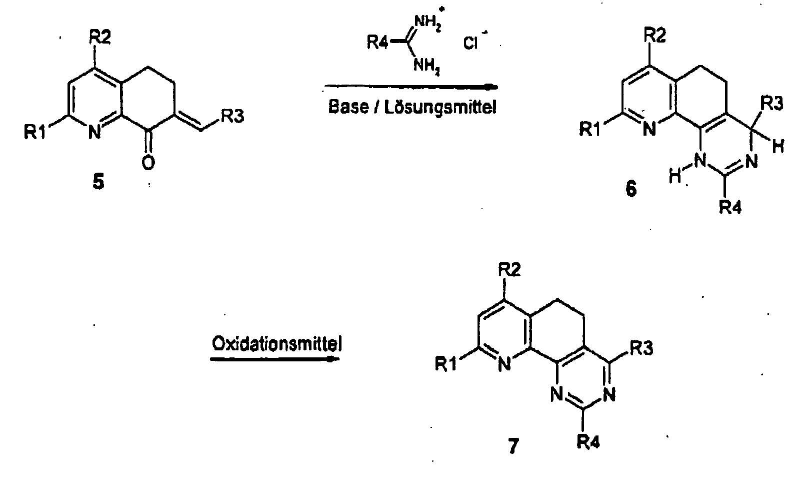
(iii) optional Aromatisierung des 5,6-Dihydro-pyrido[3,2-h]chinazolin 7 zum Pyrido[3,2-h]chinazolins **8** gemäß dem folgenden Reaktionsschema: 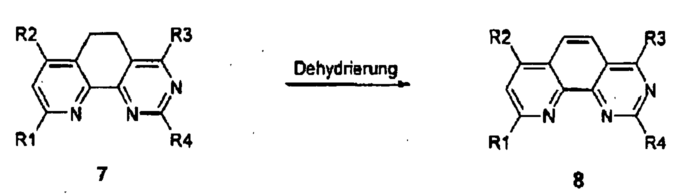
wobei R¹, R², R³ und R⁴ wie oben in Anspruch 1 definiert sind.

Dabei ist es bevorzugt, daß als Base Kaliumhydroxid und/oder Kalium-tert-butylat verwendet wird.

Auch ist bevorzugt vorgesehen, daß zur Oxidation des 1,4,5,6-Tetrahydro-pyrido[3,2-h]chinazolins 6 Chloranil eingesetzt wird.

Ebenfalls wird bevorzugt vorgeschlagen, daß die Aromatisierung durch Pd-katalysierte Dehydrierung mittels Pd/C erfolgt.

Erfindungsgemäß ist auch ein dotiertes organisches Halbleitermaterial umfassend mindestens ein organisches Matrixmaterial, das mit mindestens einem Dotanden dotiert ist, wobei das Matrixmaterial ein Pyrido[3,2-h]chinazolin und/oder 5,6-Dihydroderivat desselben ist.

Dabei ist bevorzugt, daß das Matrixmaterial reversibel reduzierbar ist.

Alternativ wird vorgeschlagen, daß das Matrixmaterial bei einer Reduktion durch den Dotanden in stabile, redox-inaktive Bestandteile zerfällt.

Der Dotand kann ein Metallkomplex sein.

Bevorzugt weist der Metallkomplex eine Struktur **I** auf, die in der Patentanmeldung DE102004010954 (entsprechend WO05086251) offenbart ist: 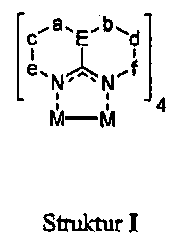 wobei M ein Übergangsmetall ist, vorzugsweise Mo oder W; und wobei
- die Strukturelemente a-f die Bedeutung: a= -CR₉R₁₀-, b = -CR₁₁R₁₂-, c = -CR₁₃R₁₄-, d = -CR₁₅R₁₆-, e = -CR₁₇R₁₈- und f = -CR₁₉R₂₀- aufweisen können, wobei R₉-R₂₀ unabhängig Wasserstoff, Alkyl mit C₁-C₂₀, Cycloalkyl mit C₁-C₂₀, Alkenyl mit C₁-C₂₀, Alkinyl mit C₁-C₂₀, Aryl, Heteroaryl, -NRR oder -OR sind, wobei R = Alkyl mit C₁-C₂₀, Cycloalkyl mit C₁-C₂₀, Alkenyl mit C₁-C₂₀, Alkinyl mit C₁-C₂₀, Aryl oder Heteroaryl ist, wobei vorzugsweise R₉, R₁₁, R₁₃, R₁₅, R₁₇, R₁₉ = H und R₁₀, R₁₂, R₁₄, R₁₆, R₁₈, R₂₀ = Alkyl mit C₁-C₂₀, Cycloalkyl mit C₁-C₂₀, Alkenyl mit C₁-C₂₀, Alkinyl mit C₁-C₂₀, Aryl, Heteroaryl, -NRR oder -OR ist, oder
- bei den Strukturelementen c und/oder d C durch Si ersetzt sein kann, oder
- wahlweise a oder b oder e oder f NR ist, mit R = Alkyl mit C₁-C₂₀, Cycloalkyl mit C₁-C₂₀, Alkenyl mit C₁-C₂₀, Alkinyl mit C₁-C₂₀, Aryl, Heteroaryl, oder
- wahlweise a und f oder b und e NR sind, mit R = Alkyl mit C₁-C₂₀, Cycloalkyl mit C₁-C₂₀, Alkenyl mit C₁-C₂₀, Alkinyl mit C₁-C₂₀, Aryl, Heteroaryl,
- wobei die Bindungen a-c, b-d, c-e und d-f, aber nicht gleichzeitig a-c und c-e und nicht gleichzeitig b-d und d-f, ungesättigt sein können,
- wobei die Bindungen a-c, b-d, c-e und d-f Teil eines gesättigten oder ungesättigten Ringsystems sein können, welches auch die Heteroelemente O, S, Se, N, P, Ge, Sn enthalten kann, oder
- die Bindungen a-c, b-d, c-e und d-f Teil eines aromatischen oder kondensierten aromatischen Ringsystems sind, welches auch die Heteroelemente O, S, Si, N enthalten kann,
- wobei das Atom E ein Hauptgruppenelement ist, bevorzugt ausgewählt aus der Gruppe C, N, P, As, Sb
- wobei das Strukturelement a-e-b wahlweise Bestandteil eines gesättigten oder ungesättigten Ringsystems ist, welches auch die Heteroelemente O, S, Se, N, P, Si, Ge, Sn enthalten kann, oder
- das Strukturelement a-E-b wahlweise Bestandteil eines aromatischen Ringsystems ist, welches auch die Heteroelemente O, S, Se, N enthalten kann.

Besonders bevorzugt ist, daß der Dotand die folgende Struktur **Ia** aufweist:

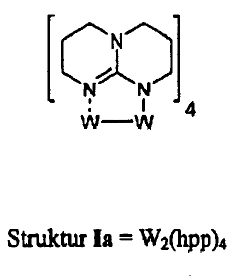

Bevorzugt ist alternativ auch, daß der Dotand eine organische Verbindung mit ausreichend hohem Oxidationspotential (HOMO) ist.

Bevorzugt ist alternativ auch, daß der Dotand ein Alkali- und/oder Erdalkalimetall ist, vorzugsweise Cäsium.

Auch wird vorgeschlagen, daß das erfindungsgemäße organische Halbleitermaterial ein Energieniveau für das unterste unbesetzte Molekülorbital (LUMO) aufweist, welches sich um 0 bis 0,5V gegenüber dem Ionisationspotential (HOMO) des Dotanden, bevorzugt um 0 bis 0,3V, besonders bevorzugt um 0 bis 0,15V, unterscheidet.

Eine Ausführungsform zeichnet sich dadurch aus, daß das Matrixmaterial ein LUMO-Energieniveau aufweist, welches tiefer liegt als das HOMO des Dotanden. "Tiefer" bedeutet in diesem Fall, dass das LUMO-Energieniveau einen kleineren Zahlenwert aufweist als das HOMO-Energieniveau. Da beide Größen negativ vom Vakuumniveau aus angegeben werden, bedeutet dies, dass der absolute Betrag des HOMO-Energiewertes kleiner ist als der absolute Betrag des LUMO-Energiewertes.

Bevorzugt ist auch, daß die Konzentration des Dotanden 0,5 bis 25 Masseprozent, bevorzugt 1 bis 15 Masseprozent, besonders bevorzugt 2,5 bis 5 Masseprozent beträgt.

Erfindungsgemäß ist auch eine organische Leuchtdiode, welche ein erfindungsgemäßes Halbleitermaterial umfaßt, sowie Benzylidenhydrochinolinone der Struktur 5.

Überraschenderweise wurde festgestellt, daß Pyrido[3,2-h]chinazoline und/oder deren 5,6-Dihydroderivate als redoxdotierbare Matrixmaterialien eingesetzt werden können, die mit Metallkomplexdotanden dotiert werden können, insbesondere mit denen der Struktur I.

Bei Einsatz solcher dotierten Schichten die Leistungseffizienz einer erfindungsgemäßen OLED erhöht.

Die erfindungsgemäß für das Halbleitermaterial eingesetzten Matrixmaterialien zeigen ferner eine gegenüber dem Stand der Technik verbesserte thermische Stabilität, die insbesondere auf erhöhte Glasübergangstemperaturen, und erhöhte Sublimations- und Zersetzungstemperaturen zurückzuführen ist.

Bevorzugt ist, dass die Glasübergangstemperatur Tg des Matrixmaterials höher ist als diejenige von 4,7-Diphenyl-1,10-phenanthrolin (Tg = 63°C).

Bevorzugt weist das Matrixmaterial eine Verdampfungstemperatur von mindestens 200°C auf.

Bevorzugt weist das Matrixmaterial eine Zersetzungstemperatur von mindestens 300°C auf.

4,6,7-Triazaphenanthrene, oder systematisch: Pyrido*[*3,2-h*]*chinazoline, wie voran stehend beschrieben, sind aus der Literatur bisher nicht bekannt.

2,4-Disubstituierte Chinolinone der Struktur 4 lassen sich in einer Dreistufensynthese, ausgehend von 2,4,6-Triphenylpyryliumsalzen **1** und Cyclohexandion, über die Stufe der 8-Oxo-1-benzopyryliumsalze **3** gewinnen (s. Schema 1). 8-Oxo-1-benzopyryliumperchlorat wurde bereits von Zimmermann et al. beschrieben. (T. Zimmermann, G.W. Fischer, B. Olk, M. Findeisen: J. Prakt. Chem. 331, 306-318 [1989]), Da die Verwendung von Perchloraten in größerem Maßstab aus Sicherheitsgründen problematisch ist, wurde in der vorliegenden Erfindung die Synthese über das Tetrafluoroborat 3 neu entwickelt.

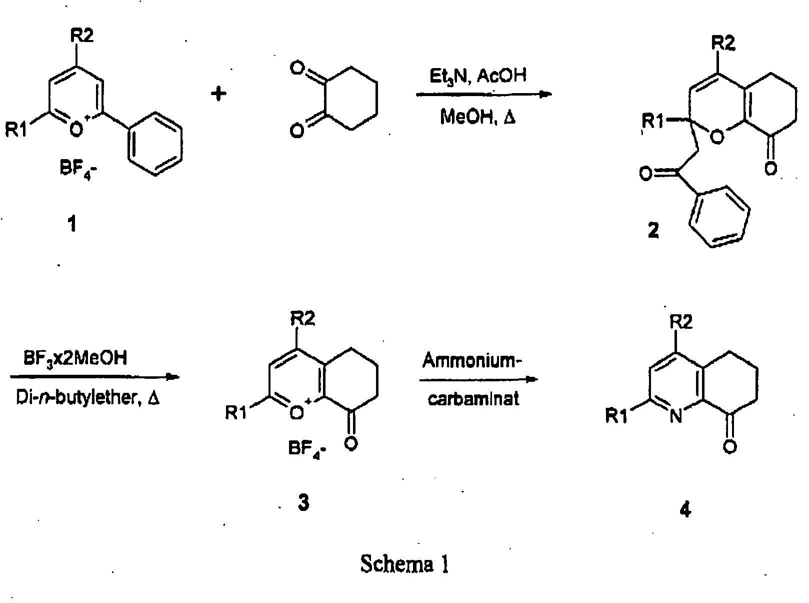

2,4-disubstituierte Chinolinone der Struktur **4** haben sich dabei als besonders wertvolle Synthesebausteine für den Aufbau von N-Heterozyklen erwiesen. Es ist bekannt, dass die Kondensation von Chinolinonen und Aldehyden zur Bildung von Benzylidenderivaten herangezogen werden kann. Die Anwendung dieser Reaktion auf die 2,4-disubstituierten Chinolinone nach Schema 2 führt zu neuen Benzylidenhydrochinolinonen **5**.

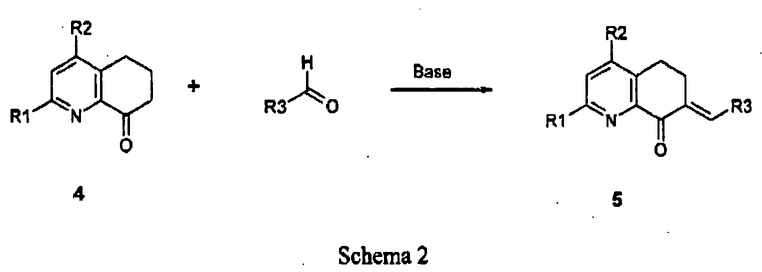

Es hat sich gezeigt, dass diese Substanzklasse die Schlüsselverbindung zur Darstellung von neuartigen Pyrido*[*3,2-h*]*chinazolinen **8** darstellt, welche Hauptgegenstand der vorliegenden Erfindung sind. Es wurde überraschend gefunden, dass die Umsetzung der genannten Benzylidendihydrochinolinone **5** mit Henzarnidiniumhydrochlorid unter basischen Bedingungen zur Bildung von Zwischenverbindungen **6** führt, welche anschließend durch Oxidation, bspw. mit Chloranil, in neuartige 5,6-Dihydro-pyrido[3,2-*h*]chinazoline **7** nach Schema 3 übergeführt werden können.

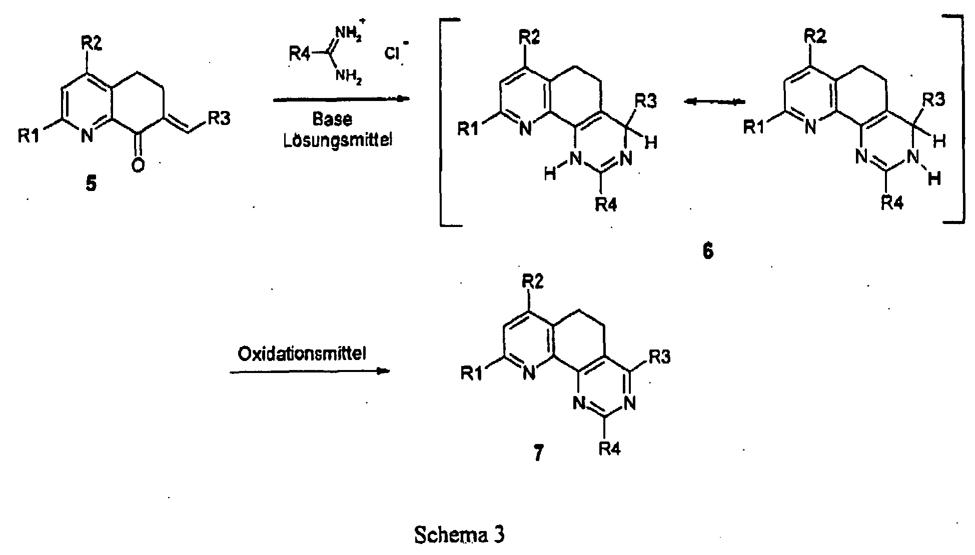

Die anschließende Aromatisierung zu neuen Pyrido*[*3,2-h*]*chinazolinen **8** kann bspw. durch Pd-katalysierte Dehydrierung der Dihydroverbindungen **7** nach Schema 4 erfolgen.

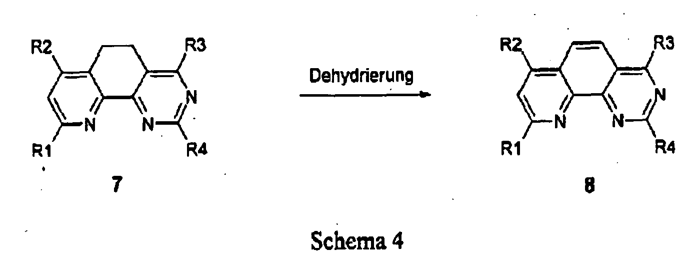

Weitere Merkmale und Vorteile der Erfindung ergeben sich aus der nachfolgenden detaillierten Beschreibung bevorzugter Ausführungsbeispiele.

### Beispiel 1

Es wird die vollständige Synthese eines Derivates **8a** der neuen Substanzklasse der 2,4,7,9-tetrasubstituierten Pyrido-*[3,2-h]*-chinazoline **8** im Folgenden beschrieben.

### Stufe 1: Synthese von 5,6-Dihydro-2-phenacyl-2,4-diphenyl-2H,7H-1-benzopyran-8-on 2a

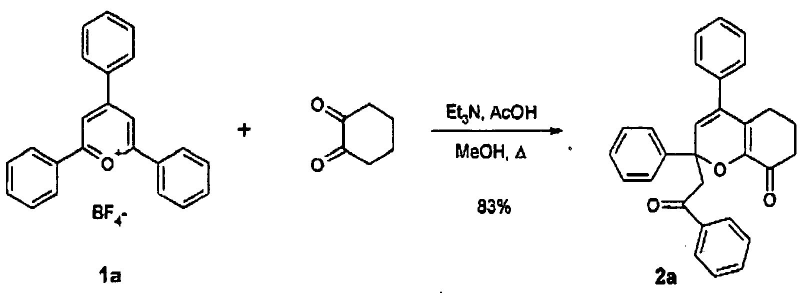

**Lit**.: T. Zimmermann, G.W. Fischer, B. Olk, M. Findeisen: J. prakt. Chem. 331, 306-318 [1989]

Eine Suspension von 13,51g 2,4,6-Triphenylpyryliurntetrafluoroborat (34,1mmol) und 4,78g Cyclohexan-1,2-dion (42,6mmol) wird in Methanol zum Sieden erhitzt, dann wird eine Lösung von 4,56g Essigsäure (75,9mmol) und 7,68g Triethylamin (75,9 mmol) in wenig Methanol hinzugetropft. Nach 7 h Erhitzen unter Rückfluss lässt man auf Raumtemperatur abkühlen. Der hellgelbe Niederschlag wird abgesaugt, portionsweise mit 100 ml eisgekühltem Methanol gewaschen und zur Gewichtskonstanz getrocknet. Man erhält 11,37 g eines hellgelben Pulvers (79,3%) mit einem Schmelzpunkt von 178 -179°C (Lit.: 182 - 184°C).

### Stufe 2: Synthese von 5,6,7,8-Tetrahydro-8-oxo-2,4-diphenyl-1-benzopyrylium-tetrafluoroborat 3a

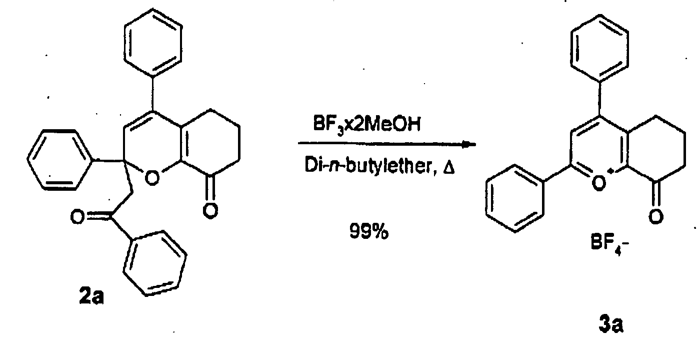

Die Suspension von 32,7g 5,6-Dihydro-2-phenacyl-2,4-diphenyl-*2H,7H*-1-benzopyran-8-on (77,8mmol) in 500ml Di-*n*-butylether wird mit 25ml BF₃-Dimethanol-Komplex (50% BF₃) versetzt, wobei sich die Suspension orange färbt. Die Suspension wird 2 h bei Raumtemperatur nachgerührt und anschließend zum Sieden erhitzt, anschließend 2 h unter Rückfluss gekocht. Nach Abkühlen auf Raumtemperatur wird der Niederschlag über eine Nutsche mit Papierfilter abgesaugt, mit Diethylether gewaschen. Nach Trocknen im Hochvakuum erhält man 29,9g (99%) terracottafarbenes Pulver mit einem Schmelzpunkt von 213-214°C (Zersetzung). IR: Die CO-Valenz-Schwingungsbande erscheint bei 1718 cm⁻¹.

### Stufe 3: Synthese von 6,7-pihydro-2,4-diphenylchinolin-8(5H)-on 4a

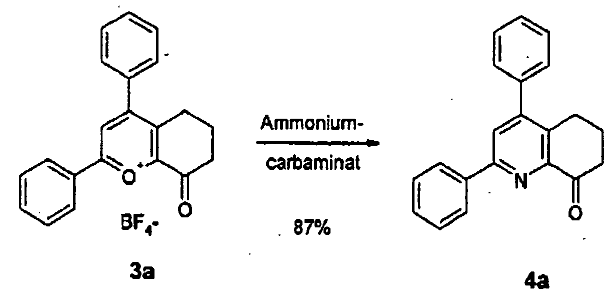

Es werden 12,44g 5,6,7,8-Tetxahydro-8-oxo-2,4-diphenyl-1-benzopyrylium-tetraflubroborat (32mmo1) in 80ml destilliertem Wasser suspendiert und unter Rühren mit einer Lösung von 25g Ammoniumcarbaminat (320mmol) in 200 ml destilliertem Wasser versetzt. Nach 24h Rühren bei Raumtemperatur wird über eine Nutsche abgesaugt, mit destilliertem Wasser gewaschen und trocken gesaugt. Das cremefarbene Pulver wird portionsweise mit Diethylether gewaschen und bei 50°C im Vakuumtrockenschrank bis zur Gewichtskonstanz getrocknet: 8,39 g elfenbeinfarbenes Produkt (87,5%) mit einem Schmelzpunkt von 177-178°C (Zersetzung). IR (ATR): Die CO-Valenz-Schwingungsbande ist bei 1700 cm⁻¹ zu sehen. ¹H-NMR (500 MHz, CDCl₃): δ = 8,09 (d, 2H, o-H von 2-Phenyl), 7,79 (s, 1H, H₃), 7,56 - 7,33 (m, 8H, arom. H), 2,92 (t, 2H, *J* = 5,75 Hz), 2,83 (t, 2H, *J* = 6,56 Hz), 2,10 (p, 2H, *J* = 5,75 Hz, *J*= 6,56 Hz).

### Stufe 4: Synthese von 7-Benzyliden-6,7-dihydro-2,4-diphenyl-chinolin-8(5H)-on 5a

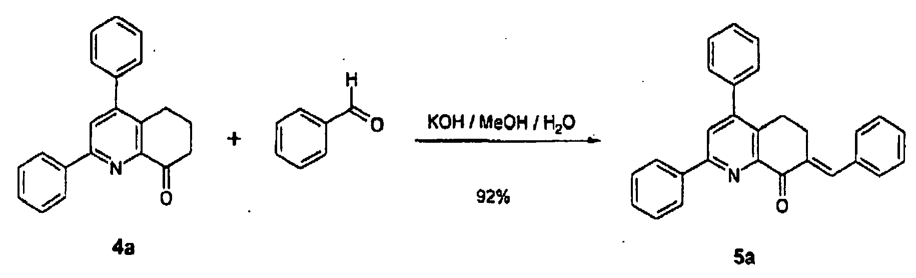

Zur Suspension von 13,8 g 6,7-Dihydro-2,4-diphenyl-chinolin-8(*5H*)-on (46 mmol) und 5,9 g Benzaldehyd (55mmol) in 200 ml Methanol wird unter gutem Rühren die Lösung von 5,6 g Kaliumhydroxid (100mmol) in 40ml destilliertem Wasser hinzugefügt. Die cremefarbene Suspension wird mit 8ml Eisessig neutralisiert und 30min bei Raumtemperatur nachgerührt. Das elfenbeinfarbene Produkt wird abgesaugt, mit 100 ml destilliertem Wasser gewaschen, scharf trocken gesaugt und luftgetrocknet. Das elfenbeinfarbene Pulver wird in 80 ml Methanol suspendiert und 30min bei Raumtemperatur nachgerührt. Der Niederschlag wird abgesaugt, mit wenig Methanol sowie Diethylether gewaschen, trocken gesaugt und bei 60°C im Vakuumtrockenschrank bis zur Gewichtskonstanz getrocknet. Man erhält 16,38g eines elfenbeinfarbenen Pulvers (91,9%) mit einem Schmelzpunkt von 185 - 187°C (Zersetzung). Das Produkt zeigt im IR Spektrum die für Chalkone charakteristische CO-Valenzschwingungsbande bei 1675 cm⁻¹. ¹H-NMR (500 MHz; CD₂Cl₂): δ = 8.13 (d, 2H, *o*-H von 2-Phenyl), 7,93 (s, 1H, H₉), 7,87 (s, 1H, H₃), 7,56 - 7,32 (m, 13 arom. H), 3,09 (dt, 2H), 2,95 (t, 2H).

### Stufe 5: Synthese von 1,4,5,6-Tetrahydro-2,4,7,9-tetraphenyl-pyrido[3,2-h]chinazolin 6a

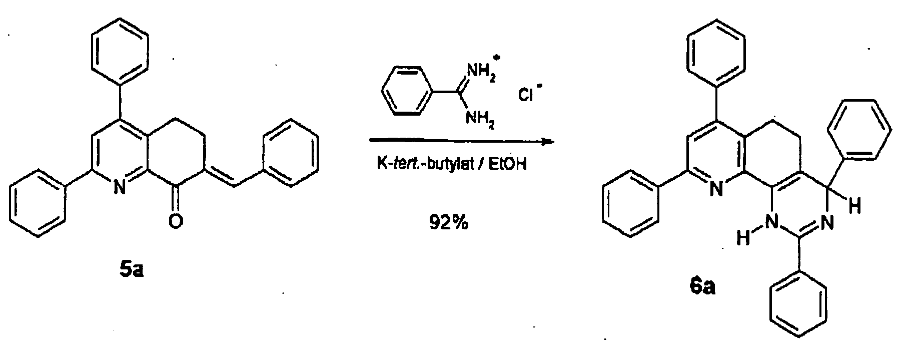

Es werden 1,71 g 7-Benzyliden-6,7-dihydro-2,4-diphenyl-chinolin-8(*5H*)-on (4.4mmol) und 0,79g Benzamidiniumchlorid-Monohydrat (4,mmol) in 30 ml Ethanol suspendiert und zum Sieden erhitzt. Zu dieser Suspension wird eine Lösung von 0,56 g Kalium-*tert*.-butylat (5mmol) in 20ml Ethanol hinzugetropft, wobei sich der Niederschlag allmählich auflöst. Nach 1,5h Kochen unter Rückfluss entsteht ein feinkristalliner, farbloser Niederschlag. Nach weiteren 16h Erhitzen unter Rückfluss wird die Suspension an der Luft auf Raumtemperatur abgekühlt. Der elfenbeinfarbene Niederschlag wird abgesaugt, mit destilliertem Wasser gewaschen und mit 30 ml Ethanol nach gewaschen. Nach trocknen im Hochvakuum erhält man 2g (92,8%) nahezu weißes, watteähnliches Pulver. mit einem Schmelzpunkt von 142-144°C (keine klare Schmelze). Aus der TGA ergibt sich ein Schmelzpunkt von 139°C, aus der DSC eine T_{g} von 74°C. Das Produkt zeigt im IR-Spektrum (ATR) eine verbreiterte NH-Bande bei 3385 cm¹ sowie in geringer Intensität eine rande bei 1687 cm⁻¹, die einer isolierten C=N Bande zuzuordnen ist. Die etwas intensivere Bande bei 1630 cm⁻¹ ist auf den Dihydrochinolinring zurückzuführen. ¹H-NMR (500 MHz, CD₂Cl₂): δ = 8,46 (s, br, NH), 8,12 - 8,04 (m, 2H, *o*-H von 9-Phenyl), 7,95 - 7,88 (m, 2H, *o*-H von 2-Phenyl), 7,60 (s, 1H, H₈), 7,56 - 7,22 (m, 16H, arom. H), 5,40 (s, 1H, H₄), 2,99 - 2,80 (m, 2H), 2,30 - 2,18 (m, 1H), 2,18 - 2,06 (m, 1H).

### Stufe 6: Synthese von 5,6-Dihydro-2,4,7,9-tetraphenyl-pyrido[3,2-h]chinazolin 7^{a}

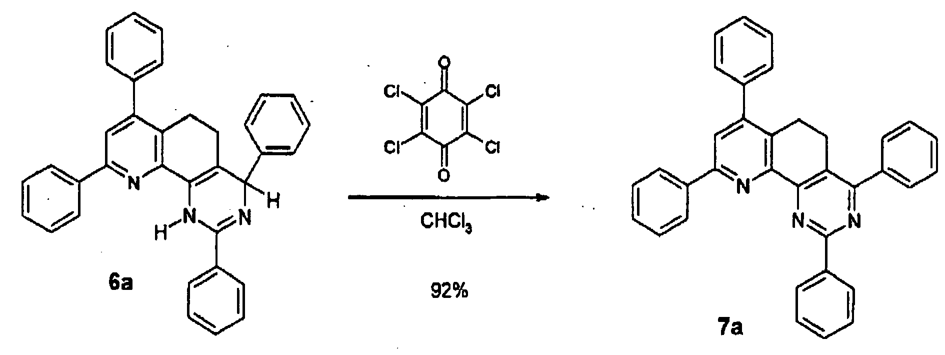

Es werden 3,43g 2,4,7,9-Texraphenyl,1,4,5,6-tetrahydropyrido[*3,2*-*h*]chinazolin (7mmol) in 50ml Chloroform gelöst; dann werden in einer Portion 1,97g Chloranil (8mmol) hinzugefügt. Die resultierende bräunliche Suspension wird bei Raumtemperatur weitergerührt. Bereits nach 30 min zeigt die DC-Kontrolle der überstehenden Lösung (Aluminiumoxid; Laufmittel: Methylenchlorid), dass der Fleck des Edukts weitgehend verschwunden ist. Die Suspension wird weitere 2h bei Raumtemperatur nachgerührt und abgesaugt und luftgetrocknet. Das cremefarbene Pulver (1,35g) wird portionsweise mit 50ml Chloroform gewaschen. Die Chloroformlösung wird mit 100ml 6%iger Kaliumcarbonatlösung versetzt und kräftig durchgerührt. Die wässrige Phase wird im Scheidetrichter abgetrennt, zweimal mit jeweils 25 ml Chloroform ausgeschüttelt und verworfen. Die vereinigten Chloroformphasen werden mit destilliertem Wasser gewaschen und nach Abtrennen der wässrigen Phase über wasserfreiem Kaliumcarbonat getrocknet. Die getrocknete hellgelbe Chloroformlösung wird über ein Faltenfilter filtriert und mit 50 ml Cyclohexan versetzt. Das Chloroform wird am Rotavapor unter vermindertem Druck weitgehend abdestilliert. Das erhaltene cremefarbene, mikrokristalline Pulver wird abgesaugt, mit Cyclohexan gewaschen und im Hochvakuum getrocknet: man erhält 3,10 g elfenbeinfarbenes, mikrokristallines Produkt (91%) mit einem Schmelzpunkt von 298 - 299°C. Die TGA zeigt den Schmelzpeak bei 299°C. Nach Abkühlen der Probe zeigt die 2. Aufheizung eine T_{g} bei 103°C. Im IR-Spektrum sind die NH-Bande bei 3385 cm⁻¹ sowie die C=N-Banden bei 1687 cm⁻¹ und 1630 cm⁻¹ des Edukts verschwunden, stattdessen ist in geringer Intensität bei 1591 cm⁻¹ resp. 1581 cm⁻¹ eine neue aufgespaltene Bande zu beobachten. ¹H-NMR (500 MHz, CDCl₃): δ = 8,78 (d, 2H, *o*-H von 2-Phenyl), 8,31 (d, 2H, *o*-H von 9-Phcnyl), 7,83 (s, 1H, H₈), 7,78-7,73 (m, 2H, *o*-H von 4-Phenyl), 7,64-7,36 (m, 14H, arom. H), 3,08 (dd, 2H), 2,95 (dd, 2H).

5,6-Dihydro-2,4,7,9-tetraphenyl-pyrido[*3,2*-*h*]chinazolin **7a** besitzt in THF das Reduktionshalbstufenpotential E_{1/2}^{l,red} = -2.27 V (ΔEₚ^{l,red} =70 mV) vs. Fc/Fc⁺. Die Reduktion ist reversibel. Ein Oxidationspotential ist innerhalb des in THF zur Verfugung stehenden Fensters nicht messbar.

### Stufe 7 Synthese von 2,4,7,9-Tetraphenyl-pyrido[3,2-h]chinazolin 8a

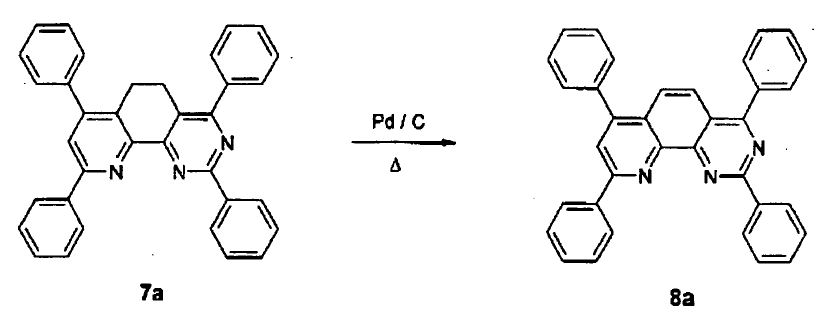

Es werden 940mg 5,6-Dihydro-2,4,7,9-tetraphenyl-pyrido[3,2-h]chinazolin (1,93mmol) in 50ml Diethylenglykol suspendiert und durch Erwärmen in Lösung gebracht, dann werden 200mg Pd / C (5%) portionsweise hinzugefügt. Die Suspension wird zum Sieden erhitzt, 10 h unter Rückfluss erhitzt und anschließend an der Luft auf Raumtemperatur abgekühlt. Nach DC Kontrolle werden 50 ml destilliertes Wasser hinzugefügt. Die Suspension wird 30 min bei Raumtemperatur nachgerührt, filtriert und der Filterkuchen portionsweise mit destilliertem Wasser gewaschen und scharf trockengesaugt. Der mit weißen Kristallen durchsetzte schwarze Rückstand wird auf der Glasfritte portionsweise mit 400 ml Chloroform gewaschen. Das bräunlich-gelbe Filtrat wird über wasserfreiem Natriumsulfat getrocknet, filtriert und bis zur Trockene eingedampft. Der cremefarbene Rückstand wird in 50 ml Chloroform unter leichtem Erwärmen gelöst und in 100 ml Cyclohexan filtriert. Die hellgelbe Lösung wird auf das halbe Volumen eingeengt, wobei sich ein elfenbeinfarbener, watteähnlicher Niederschlag abscheidet. Dieser wird abgesaugt, mit wenig Cyclohexan sowie Diethylether gewaschen und im Hochvakuum getrocknet: man erhält 0,82g (87,5%) eines mikrokristallinen, weißen Pulvers mit sehr scharfem Schmelzpunkt bei 327°C. Eine Glasübergangstemperatur T_{g} ist zunächst nicht messbar. Die kontrolliert aufgenommene Abkühlkurve (10 K/min) nach der 2. Aufheizung zeigt Rekristallisation bei 272 °C. Nach schnellem Abkühlen wird in der DSC eine T_{g} von 103°C gemessen. Das ¹H-NMR-Spektrum enthält nur die Signale von 5,6-Dihydro-2,4,7,9-tetraphenyl-pyrido[*3*,*2*-*h*]chinazolin. Die Signale der Methylenprotonen des Eduktes bei 3.07 (dd, 2H) und 2.95 (dd, 2H) sind verschwunden.

Tetraphenyl-pyrido*[3*,*2*-*h]*chinazolin **8a** besitzt in MeCN das Reduktionshalbstufenpotential E_{1/2}^{I,red} = -2.06 V (ΔEₚ^{I,red} = 70 mV) vs. Fc/Fc⁺. Die Reduktion ist reversibel. Ein Oxidationspotential ist innerhalb des in MeCN zur Verfügung stehenden Fensters nicht messbar.

Eine Zersetzungstemperatur von **8a** (DSC) unter Stickstoff konnte bis 500°C nicht festgestellt werden. Somit ist die Verbindungsklasse thermisch stark belastbar. Die Sublimationstemperatur von **8a** beträgt 259°C und befindet sich damit deutlich über der 200°C Marke.

Aus einem Vergleich der Glasübergangstemperaturen von **7a** und **8a** mit BPhen ergibt sich, dass die morphologische Stabilität von BPhen sehr gering, hingegen die von **7a** und **8a** deutlich erhöht ist.

### Beispiel 2

Im Folgenden wird die Teilsynthese eines Derivates **8b** ausgehend von **4a** beschrieben.

### Stufe 4: Synthese von 7-Toluyden-6,7-dihydro-2,4-diphenyl-chinolin-8(5H)-on 5b

Zu einer Suspension von 5 g 6,7-Dihydro-2,4-diphenyl-chinolin-8(*5H*)-on (16.7 mmol) und 2.41g (~ 2.4ml) p-Methylbenzaldehyd (20.04mmol) in 40 ml Methanol wird wie im Beispiel 1 beschrieben eine Lösung von 1,5 g Kaliumhydroxid (36,7mmol) in 15ml destilliertem Wasser hinzugefügt. Die resultierende Suspension wird bei Raumtemperatur im verschlossenen Rundkolben 1d gerührt, mit Eisessig neutralisiert und 30 min bei Raumtemperatur nachgerührt. Der Niederschlag wird abgesaugt, mit destilliertem Wasser gewaschen und getrocknet. Das elfenbeinfarbene Pulver wird in 80 ml Methanol suspendiert und 30 min bei Raumtemperatur nachgerührt. Der Niederschlag wird abgesaugt, mit Methanol sowie Diethylether gewaschen und im Hochvakuum getrocknet: 3.2 g elfenbeinfarbenes Pulver (47 %) mit einem Schmelzpunkt von 173 - 1.85°C (Zersetzung). Das Produkt zeigt die für Chalkone charakteristische CO-Valenzschwingungsbande bei 1675 cm⁻¹.
¹H-NMR (500 MHz; CD₂Cl₂): δ = 8,13 (d, 2H, o-H von 2-Phenyl), 7,96 (s, 1H, H₉), 7,81 (s, 1H, H₃), 7,56 - 7,32 (m, 11 arom. H), 7,21 (d, 2H von Phenyl), 3,08 (dt, 2H), 2,94 (t, 2H), 2,37 8s, 3H.

### Stufe 5: Synthese von 1,4,5,6-Tetrahydro-2,7,9-triphenyl-4-p-toluyl-pyrido[3,2-h]chinazolin 6b

Es werden 3g 7-Toluyden-6,7-dihydro-2,4-diphenyl-chinolin-8(*5H*)-on (7,5mmol) und 1,6g Benzamidiniumchlorid-Monohydrat (8,2mmol) in 50ml Ethanol suspendiert und zum Sieden erhitzt. Zu dieser Suspension wird eine Lösung von 1,2 g Kalium-*tert*.-butylat (10 mmol) in 20 ml Ethanol hinzugetropft, wobei sich der Niederschlag allmählich auflöst. Nach 24 h Kochen unter Rückfluss lässt man die Suspension auf Raumtemperatur abkühlen. Der Niederschlag wird abgesaugt, mit destilliertem und Ethanol gewaschen und im Hochvakuum bis zur Gewichtskonstanz getrocknet. Man erhält 2,6g Produkt (57%) mit einem Schmelzpunkt von 128-133°C.

Das Produkt zeigt im IR-Spektrum (ATR) eine verbreiterte NH-Bande bei 3418 cm¹ sowie in geringer Intensität eine Bande bei 1625 cm⁻¹, die einer isolierten C=N Bande zuzuordnen ist.
¹H-NMR (500 MHz, CD₂Cl₂): δ = 8,43 (s, br, NH), 8,09 (d, 2H, *o*-H von 9-Phenyl), 7,93-7,91 (m, 2H, *o*-H von 2-Phenyl), 7,59 (s, 1H, H₈), 7,54 - 7,43 (m, 10H, arom. H), 7,38 (d, 2H), 7,35 (d, 2H), 7,15 (d, 2H), 5,35 (s, 1H, H₄), 2,95 - 2,82 (m, 2H), 2,32 (s, 3H), 2,26 - 2,20 (m, 1H), 2,15 - 2,08 (m, 1H).

### Stufe 6b: Synthese von 5,6-Dihydro-2,7,9-triphenyl-4-p-toluyl-pyrido[3,2-h]chinazolin 7b

Es werden 2,5g 2,4,7,9-Tetraphenyl-1,4,5,6-tetrahydropyrido[*3,2*-*h*]chinazolin (4,96mmol) in 70 ml Chloroform gelöst; dann werden in einer Portion 1.46g Chloranil (5,95mmol) hinzugefügt. Die resultierende bräunliche Suspension wird bei Raumtemperatur weitergerührt. Bereits nach 30min zeigt die DC-Kontrolle der überstehenden Lösung (Aluminiumoxid; Laufmittel: Methylenchlorid), dass die Umsetzung vollständig ist. Die Suspension wird über eine D4-Glasfritte filtriert. Die Chloroformlösung wird mit 100 ml 6%iger Kaliumcarbonatlösung versetzt und kräftig durchgerührt. Die wässrige Phase wird zweimal mit jeweils 25 ml Chloroform ausgeschüttelt und dann verworfen. Die vereinigten Chloroformphasen werden mit destilliertem Wasser gewaschen, über wasserfreiem Kaliumcarbonat getrocknet, filtriert und mit 50 ml Cyclohexan versetzt. Das Chloroform wird unter vermindertem Druck weitgehend abdestilliert. Das erhaltene Pulver wird abgesaugt, mit etwa wenig Cyclohexan gewaschen und im Hochvakuum getrocknet; man erhält 2,1g (85%) elfenbeinfarbenes, mikrokristallines Produkt mit einem Schmelzpunkt von 261°C und einer Tg von 109°C. ¹H-NMR (500 MHz, CDCl₃): δ = 8,77 (d, 2H, *o*-H von 2-Phenyl), 8,29 (d, 2H, *o*-H von 9-Phenyl), 7,81 (s, 1H, H₈), 7,67 (d, 2H, *o*-H von 4-Phenyl), 7,56 - 7,44 (m, 10H, arom. H), 7,42 (d, 2H, Phenyl), 7,32 (d, 2H, Phenyl), 3,07 (m, 2H), 2,93 (m, 2H), 2,44 (s, 3H).

5,6-Dihydro-2,7,9-triphenyl-4-*p*-toluyl-pyrido[3,2-*h*]chinazolin 7b besitzt in THF das Reduktionshalbstufen -potential E_{1/2}^{l,red} = -2.29 V (ΔEₚ^{l,red} =70 mV) vs. Fc/Fc⁺. Die Reduktion ist reversibel. Ein Oxidationspotential ist innerhalb des in THF zur Verfügung stehenden Fensters nicht messbar.

### Stufe 7: Synthese von 2,7,9-Triphenyl-4-p-toluylpyrido[3,2-h]chinazolin 8b

Es werden 2g 5,6-Dihydro-2,7,9-triphenyl-4-*p*-toluyl-pyrido[*3*,*2*-*h*]chinazolin (3.98mmol) in 100ml Diethylenglykol suspendiert und durch Erwärmen in Lösung gebracht, dann werden 500 mg Pd / C (10%) portionsweise hinzugefügt. Die Suspension wird zum Sieden erhitzt und unter Rückfluss gekocht, bis laut DC Kontrolle die Ausgangsverbindung nicht mehr nachweisbar ist. Es werden 50ml destilliertes Wasser hinzugefügt, 30min bei Raumtemperatur nachgerührt und filtriert. Der Rückstand wird portionsweise mit 300ml destilliertem Wasser gewaschen und trocken gesaugt. Dann wird der Rückstand portionsweise mit 600ml Chloroform gewaschen. Das Filtrat wird über wasserfreiem Natriumsulfat getrocknet und filtriert. Die Lösung wird unter leicht vermindertem Druck zur Trockene eingedampft. Der cremefarbene Rückstand wird in 50ml Chloroform unter leichtem Erwärmen gelöst und in 100ml Cyclohexan filtriert. Die hellgelbe Lösung wird unter vermindertem Druck auf das halbe Volumen eingeengt, wobei sich ein elfenbeinfarbener, watteähnlicher Niederschlag abscheidet. Der Niederschlag wird abgesaugt, mit Cyclohexan sowie Diethylether gewaschen und im Hochvakuum getrocknet. Man erhält 1,5g (75%) mikrokristallines, weißes, watteähnliches Pulver mit einer Schmelztemperatur von 261°C. Eine Tg konnte mittels DSC nicht detektiert werden.

Das ¹H-NMR-Spektrum in CDCl₃ enthält nur die Signale von 5,6-Dihydro-2,4,7,9-tetraphenyl-pyrido[*3,2-h*]chinazolin. Die Signale der Methylenprotonen des Eduktes bei 3,07 ( dd, 2H) und 2,95 (dd, 2H) sind verschwunden, d.h., die Dehydrierung ist vollständig verlaufen.

2,7,9-Triphenyl-4-*p*-toluylpyrido[*3*,*2*-*h*]chinazolin **8b** besitzt in THF das Reduktionshalbstufenpotential E_{1/2}^{l,red}= -2.29V (ΔEₚ^{l,red} = 70 mV) *vs* Fc/Fc⁺. Die Reduktion ist reversibel. Ein Oxidationspotential ist innerhalb des in THF zur Verfügung stehenden Fensters nicht messbar.

Eine Zersetzungstemperatur von **8b** (DSC) unter Stickstoff konnte bis 500°C nicht festgestellt werden. Somit ist die Verbindungsklasse thermisch stark belastbar. Die Sublimationstemperatur von **8b** beträgt 272°C und befindet sich damit deutlich über der 200°C Marke.

Aus einem Vergleich der Glasübergangstemperaturen von **7b** und **8b** mit **BPhen** ergibt sich, dass die morphologische Stabilität von BPhen sehr gering, hingegen die von **7a** und **8a** deutlich erhöht ist.

### Beispiel 3

Ein Glassubstrat wird mit Kontakten aus Indium-Zinn-Oxid versehen. Auf das Substrat wird danach eine Schicht aus **7a** dotiert mit einem Dotanden der Struktur **Ia** hergestellt. Die Dotierkonzentration des Dotanden **Ia** beträgt 5mol%. Die Leitfähigkeit der Schicht beträgt 4.13*10⁻⁵ S/cm bei Raumtemperatur.

### Beispiel 4

Ein Glassubstrat wird mit Kontakten aus Indium-Zinn-Oxid versehen. Auf das Substrat wird danach eine Schicht aus **8a** dotiert mit einem Dotanden der Struktur **Ia** hergestellt. Die Dotierkonzentration des Dotanden **Ia** beträgt 5mol%. Die Leitfähigkeit der Schicht beträgt 1,84*10⁻⁵ S/cm bei Raumtemperatur.

### Beispiel 5

Ein Glassubstrat wird mit Kontakten aus Indium-Zinn-Oxid versehen. Auf das Substrat wird danach eine Schicht aus **7b** dotiert mit einem Dotanden der Struktur **Ia** hergestellt. Die Dotierkonzentration des Dotanden **Ia** beträgt 5mol%. Die Leitfähigkeit der Schicht beträgt 2.05*10⁻⁶ S/cm bei Raumtemperatur.

### Beispiel 6

Ein Glassubstrat wird mit Kontakten aus Indium-Zinn-Oxid versehen. Auf das Substrat wird danach eine Schicht aus **8b** dotiert mit einem Dotanden der Struktur **Ia** hergestellt. Die Dotierkonzentration des Dotanden **Ia** beträgt 5mol%. Die Leitfähigkeit der Schicht beträgt 2.76*10⁻⁵ S/cm bei Raumtemperatur.

### Vergleichsbeispiel 7

Ein Vergleichsbeispiel 7 wurde analog zur Vorgehensweise nach Beispielen 3 bis 6 durchgeführt, jedoch wurde anstelle des Matrixmaterials **7a, 8a** bzw. **7b, 8b** das aus der Literatur bekannte **BPhen** verwendet. Die erhaltene Leitfähigkeit der Schicht beträgt 4* 10⁻⁹ S/cm.

Ein Vergleich der Leitfähigkeiten aus den Beispielen 3 bis 6 mit den Leitfähigkeitswerten für die aus dem Stand der Technik bekannten Matrixmaterialien Alq₃, BPhen oder BAlq₂ (Tabelle 1 und Vergleichsbeispiel 7) zeigt somit für die erfindungsgemäße Substanzklasse 8 eine deutliche, um 3-4 Größenordnungen verbesserte Leitfähigkeit.

### Beispiel 8

Ein Substrat aus Glas wird mit Indium-Zinn-Oxid-Kontakten versehen. Darauf werden dann nacheinander die Schichten: Spiro-TTB dotiert mit p-Dotand 2-(6-Dicyanomethylene-1,3,4,5,7,8-hexafluoro-6H-naphthalen-2-yliden)-malononitril (50nm, 1,5 w%) / N,N'-Di(naphthalen-2-yl)-N,N'-diphenylbenzidin (10nm), N,N'-Di(naphthalen-2-yl)-N,N'-diphenylbenzidin dotiert mit Emitter Iridium(III)bis(2-methyldibenzo-[f,h]quinoxalin)(acetylacetonat) (20nm, 10wt%) / 2,4,7,9-Tetraphenylphenanthrolin (10nm) / **8a** dotiert mit **Ia** (60nm, 8 w%) / Al (100nm) abgeschieden. Die so hergestellte organische Leuchtdiode emittiert orangerotes Licht und weist bei 1000 cd/m2 folgende Betriebsparameter auf: 3,2V und 20 cd/A.

### Vergleichsbeispiel 9

### (Stand der Technik)

Eine OLED Struktur wie im Beispiel 6 wurde hergestellt. Dabei wurde statt der mit **Ia** n-dotierten Elektroncntransportschicht **8a** die undotierte Elektronentransportschicht **8a** verwendet. Diese OLED weist bei 100 cd/m2 folgende Betriebsparameter auf: 18.2V und 7.4 cd/A.

Die Verwendung von mit **Ia** dotiertem **8a** als Elektronentransportschicht führt also dazu, dass eine OLED Struktur eine geringere Betriebsspannung und/ oder eine höhere Stromeffizienz aufweist. Mithin ist auch die Leistungseffizienz des Bauelementes erhöht.

Die in der Beschreibung und in den Ansprüchen offenbarten Merkmale können sowohl cinzeln als auch in einer beliebigen Kombination zur Verwirklichung der Erfindung in ihren unterschiedlichen Ausführungsformen wesentlich sein.

## Patentansprüche

1. Pyrido[3,2-h]chinazoline der folgenden Struktur 8: 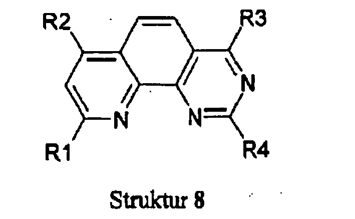 und/oder deren 5,6-Dihydroderivate
wobei:
R^{I} und R², substituiert oder unsubstituiert, Aryl, Heteroaryl, Alkyl der Formel CHR₂ mit R=Alkyl mit C₁-C₂₀ oder Alkyl der Formel CR₃ mit R=Alkyl mit C₁-C₂₀ ist;
R³ ausgewählt ist aus H, substituiert oder unsubstituiert, Alkyl mit C₁-C₂₀, Aryl und Heteroaryl;
R⁴ ausgewählt ist aus H, substituiert oder unsubstituiert, Alkyl mit C₁-C₂₀, Aryl, Heteroaryl, NH₂, NHR mit R=Alkyl mit C₁-C₂₀, NR₂ mit R=Alkyl mit C₁-C₂₀, N-Alkylaryl, N-Aryl₂, Carbazolyl, Dibenzazepinyl und CN.

2. Verfahren zur Herstellung von Pyrido[3,2-h]chinazolinen und/oder deren 5,6-Dihydroderivaten, welches die folgenden Schritte umfaßt:
(i) Umsetzung eines 2,4-disubstituierten Chinolinons der Struktur 4 mit einem Aldehyd in Gegenwart einer Base zur Darstellung eines Benzylidendihydrochinolinons 5 gemäß dem folgenden Reaktionsschema: 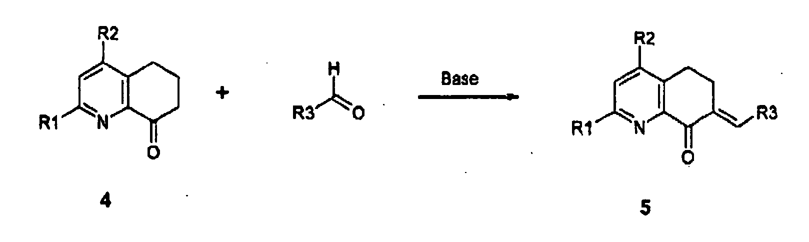
(ii) Umsetzung des Benzylidendihydrochinolinons 5 mit Benzamidiniumhydrochlorid unter basischen Bedingungen zur Darstellung eines 1,4,5,6-Tetrahydro-pyrido[3,2-h]chinazolins 6 und anschließender Oxidation zur Darstellung eines 5,6-Dihydro-pyrido[3,2-h]chinazolins 7 gemäß dem folgenden Reaktionsschema: 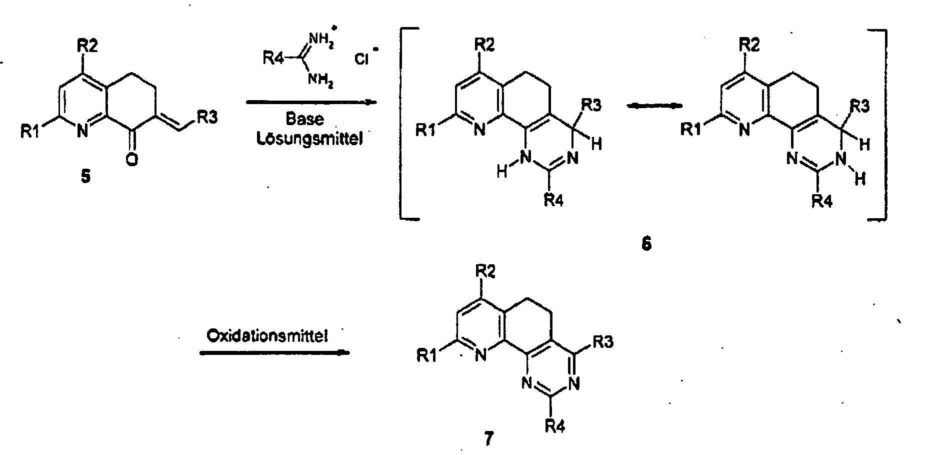
(iii) optional Aromatisierung des 5,6-Dihydro-pyrido[3,2-h]chinazolins 7 zum Pyrido[3,2-h]chinazolin 8 gemäß dem folgenden Reaktionsschema: 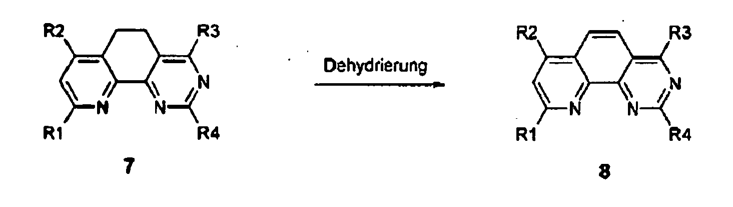 wobei R¹, R², R³ und R⁴ wie oben in Anspruch 1 definiert sind.

3. Verfahren nach Anspruch 2, **dadurch gekennzeichnet, daß** als Base Kaliumhydroxid und/oder Kalium-tert-butylat verwendet wird.

4. Verfahren nach Anspruch 2 oder 3, **dadurch gekennzeichnet, daß** zur Oxidation des 1,4,5,6-Tetrahydro-pyrido[3,2-h]chinazolins 6 Chloranil eingesetzt wird.

5. Verfahren nach einem der Ansprüche 2 bis 4, **dadurch gekennzeichnet, daß** die Aromatisierung durch Pd-katalysierte Dehydrierung mittels Pd/C erfolgt.

6. Dotiertes organisches Halbleitermaterial umfassend mindestens ein organisches Matrixmaterial, das mit mindestens einem Dotanden dotiert ist, wobei das Matrixmaterial ein Pyrido[3,2-h]chinazolin und/oder 5,6-Dihydroderivat nach Anspruch 1 ist.

7. Halbleitermaterial nach Anspruch 6, **dadurch gekennzeichnet, daß** das Matrixmaterial reversibel reduzierbar ist.

8. Halbleitermaterial nach Anspruch 6, **dadurch gekennzeichnet, daß** das Matrixmaterial bei einer Reduktion in stabile, redox-reaktive Bestandteils zerfällt.

9. Halbleitermaterial nach einem der Ansprüche 6 bis 8, **dadurch gekennzeichnet, daß** der Dotand ein Metallkomplex ist.

10. Halbleitermaterial nach Anspruch 9, **dadurch gekennzeichnet, daß** der Metallkomplex eine Struktur I aufweist: 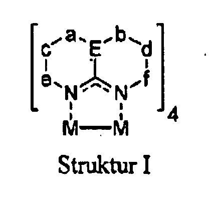 wobei M ein Übergangsmetall ist; und wobei
- die Strukturelemente a-f die Bedeutung: a= -CR₉R₁₀-, b = -CR₁₁R₁₂-, c = - CR₁₃R₁₄-, d = -CR₁₅R₁₆-, e = -CR₁₇R₁₈- und f = -CR₁₉R₂₀- aufweisen können, wobei R₉-R₂₀ unabhängig Wassentoff, Alkyl mit C₁-C₂₀, Cycloalkyl mit C₁-C₂₀, Alkenyl mit C₁-C₂₀, Alkinyl mit C₁-C₂₀, Aryl, Heteroaryl,-NRR oder -OR sind, wobei R = Alkyl mit C₁-C₂₀, Cycloalkyl mit C₁-C₂₀, Alkenyl mit C₁-C₂₀, Alkinyl mit C₁-C₂₀, Aryl oder Heteroaryl ist, oder
- bei den Strukturelementen c und/oder d C durch Si ersetzt sein kann, oder
- wahlweise a oder b oder e oder f NR ist, mit R = Alkyl mit C₁-C₂₀, Cycloalkyl mit C₁-C₂₀, Alkenyl mit C₁-C₂₀, Alkinyl mit C₁-C₂₀, Aryl, Heteroaryl, oder
- wahlweise a und f oder b und e NR sind, mit R = Alkyl mit C₁-C₂₀, Cycloalkyl mit C₁-C₂₀, Alkenyl mit C₁-C₂₀, Alkinyl mit C₁-C₂₀, Aryl, Heteroaryl,
- wobei die Bindungen a-c, b-d, c-e und d-f, aber nicht gleichzeitig a-c und c-e und nicht gleichzeitig b-d und d-f, ungesättigt sein können,
- wobei die Bindungen a-c, b-d, c-e und d-f Teil eines gesättigten oder ungesättigten Ringsystems sein können, welches auch die Heteroelemente 0, S, Se, N, P, Ge, Sn enthalten kann, oder
- die Bindungen a-c, b-d, c-e und d-f Teil eines aromatischen oder kondensierten aromatischen Ringsystems sind, welches auch die Heteroelemente 0, S, Si, N enthalten kann,
- wobei das Atom E ein Hauptgruppenelement ist,
- wobei das Strukturelement a-E-b wahlweise Bestandteil eines gesättigten oder ungesättigen Ringsystems ist, welches auch die Heteroelemente O, S, Se, N, P, Si, Ge, Sn enthalten kann, oder
- das Strukturelement a-E-b wahlweise Bestandteil eines aromatischen Ringsystems ist, welches auch die Heteroelemente O, S, Se, N enthalten kann.

11. Halbleitermaterial nach Anspruch 10, **dadurch gekennzeichnet, daß** M Mo oder W ist.

12. Halbleitermaterial nach Anspruch 10, **dadurch gekennzeichnet, daß** R₉, R₁₁, R₁₃, R₁₅, R₁₇, R₁₉ = H und R₁₀, R₁₂, R₁₄, R₁₆, R₁₈, R₂₀ = Alkyl mit C₁-C₂₀, Cycloalkyl mit C₁-C₂₀, Alkenyl mit C₁-C₂₀, Alkinyl mit C₁-C₂₀, Aryl, Heteroaryl, -NRR oder -OR ist.

13. Halbleitermaterial nach Anspruch 10, **dadurch gekennzeichnet, daß** E ausgewählt ist aus der Gruppe C, N, P, As, Sb.

14. Halbleitermaterial nach Anspruch 10, **dadurch gekennzeichnet, daß** der Dotand die folgender Struktur Ia aufweist:

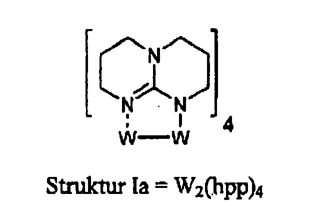

15. Halbleitermaterial nach einem der Ansprüche 6 bis 8, **dadurch gekennzeichnet, daß** der Dotand ein Alkali- und/oder Erdalkalimetall ist.

16. Halbleitermaterial nach Anspruch 15, **dadurch gekennzeichnet, daß** der Dotand Caesium ist.

17. Halbleitermaterial nach einem der vorangehenden Ansprüche, **dadurch gekennzeichnet, daß** das Matrixmaterial ein Energieniveau für das unterste unbesetzte Molekülorbital (LUMO) aufweist, welches sich um 0-0,5 V gegenüber dem Ionisationspotential (HOMO) des Dotanden unterscheidet.

18. Halbleitermaterial nach Anspruch 17, **dadurch gekennzeichnet, daß** sich das Energieniveau des Matrixmaterials gegenüber dem Ionisationspotential des Dotanden um 0-0,3 V unterscheidet.

19. Halbleitermaterial nach Anspruch 17, **dadurch gekennzeichnet, daß** sich das Energieniveau des Matrixmaterials gegenüber dem Ionisationspotential des Dotanden um 0-0,15 V unterscheidet.

20. Halbmaterial nach einem der Ansprüche 6 bis 19, **dadurch gekennzeichnet, daß** das Matrixmaterial ein LUMO-Energieniveau aufweist, welches tiefer liegt als das Ionisationspotential (HOMO) des Dotanden.

21. Halbleitermaterial nach einem der vorangehenden Ansprüche 6 bis 20, **dadurch gekennzeichnet, daß** die Konzentration des Dotanden 0,5 bis 25 Masseprozent.

22. Halbleitermaterial nach Anspruch 21, **dadurch gekennzeichnet, daß** die Konzentration des Dotanden 1 bis 10 Masseprozent beträgt.

23. Halbleitermaterial nach Anspruch 21, **dadurch gekennzeichnet, daß** die Konzentration des Dotanden 2,5 bis 5 Masseprozent beträgt.

24. Halbleitermaterial nach einem der vorangehenden Ansprüche 6 bis 23, **dadurch gekennzeichnet, daß** die Glasübergangstemperatur Tg des Matrixmaterials höher ist als diejenige von 4,7-Diphenyl-1,10-phenantrolin.

25. Halbleitermaterial nach einem der vorangehenden Ansprüche 6 bis 24, **dadurch gekennzeichnet, daß** das Matrixmaterial eine Verdampfungstemperatur von mindestens 200°C aufweist.

26. Organische Leuchtdiode, welche ein Halbleitermaterial nach einem der vorangehenden Ansprüche 6 bis 25 umfaßt.

27. Benzylidenhydrochinolinon der Formel 5 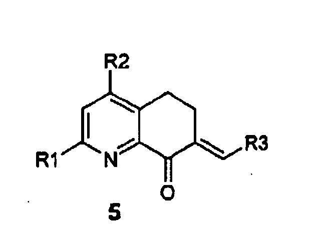 wobei R¹, R² und R³ wie in Anspruch 1 definiert sind.

## Claims

1. Pyrido[3,2-h]quinazolines of the following structure 8: 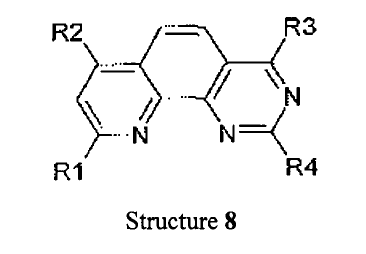 and/or 5,6-dihydro derivatives thereof
wherein:
R¹ and R², substituted or unsubstituted, are aryl, heteroaryl, alkyl of the formula CHR₂ with R = alkyl with C₁-C₂₀, or alkyl of the formula CR₃ with R = alkyl with C₁-C₂₀;
R³ is selected from H, substituted or unsubstituted, alkyl with C₁-C₂₀, aryl and heteroaryl;
R⁴ is selected from H, substituted or unsubstituted, alkyl with C₁-C₂₀, aryl, heteroaryl, NH₂, NHR with R = alkyl with C₁-C₂₀, NR₂ with R = alkyl with C₁-C₂₀, N-alkylaryl, N-aryl₂, carbazolyl, dibenzazepinyl and CN.

2. Method for the production of pyrido[3,2-h]quinazolines and/or 5,6-dihydro derivatives thereof, comprising the following steps:
(i) reaction of a 2,4-disubstituted quinolinone of structure 4 with an aldehyde in the presence of a base to prepare a benzylidene hydroquinolinone 5 in accordance with the following reaction scheme: 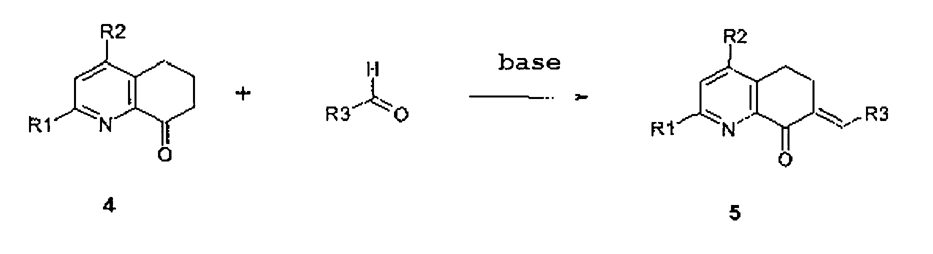
(ii) reaction of the benzylidene hydroquinolinone 5 with benzamidinium hydrochloride under basic conditions to prepare a 1,4,5,6-tetrahydropyrido[3,2-h]quinazoline 6 and subsequent oxidation to prepare a 5,6-dihydropyrido[3,2-h]quinazoline 7 in accordance with the following reaction scheme: 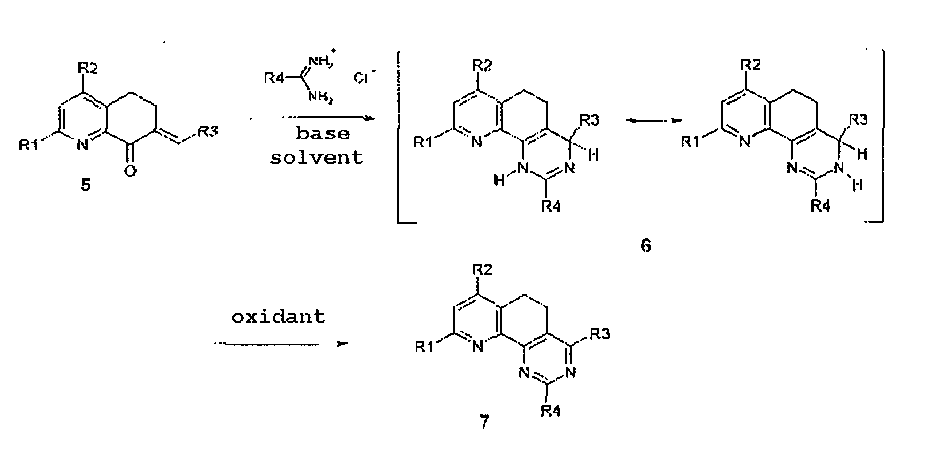
(iii) optionally aromatisation of the 5,6-dihydropyrido[3,2-h]quinazoline 7 to give the pyrido[3,2-h]quinazoline 8 in accordance with the following reaction scheme: 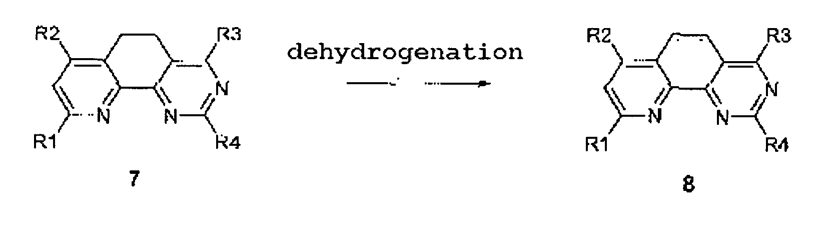 wherein R¹, R², R³ and R⁴ are defined as above in Claim 1.

3. Method according to Claim 2, **characterised in that** potassium hydroxide and/or potassium tert-butylate is used as the base.

4. Method according to Claim 2 or 3, **characterised in that** chloranil is employed for the oxidation of the 1,4,5,6-tetrahydropyrido[3,2-h]quinazoline 6.

5. Method according to one of Claims 2 to 4, **characterised in that** the aromatisation is taking place by Pd-catalysed dehydrogenation with Pd/C.

6. Doped organic semiconductor material, comprising at least one organic matrix material which is doped with at least one dopant, wherein the matrix material is a pyrido[3,2-h]quinazoline and/or 5,6-dihydro derivative thereof according to Claim 1.

7. Semiconductor material according to Claim 6, **characterised in that** the matrix material can be reversibly reduced.

8. Semiconductor material according to Claim 6, **characterised in that** the matrix material is breaking down into stabile, redox reactive components during a reduction.

9. Semiconductor material according to one of Claims 6 to 8, **characterised in that** the dopant is a metal complex.

10. Semiconductor material according to Claim 9, **characterised in that** the metal complex has a structure I: 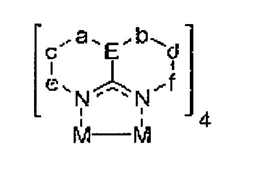 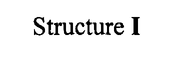 wherein M is a transition metal; and wherein
- the structure elements a - f can have the meaning: a = -CR₉R₁₀-, b = - CR₁₁R₁₂-, c = -CR₁₃R₁₄-, d = -CR₁₅R₁₆-, e = -CR₁₇R₁₈- and f = -CR₁₉R₂₀-, wherein R₉-R₂₀ are independently hydrogen, alkyl with C₁-C₂₀, cycloalkyl with C₁-C₂₀, alkenyl with C₁-C₂₀, alkinyl with C₁-C₂₀, aryl, heteroaryl, -NRR or -OR, wherein R = alkyl with C₁-C₂₀, cycloalkyl with C₁-C₂₀, alkenyl with C₁-C₂₀, alkinyl with C₁-C₂₀, aryl or heteroaryl, or
- in the structure elements c and/or d, C can be replaced by Si, or
- optionally a or b or e or f is NR, with R = alkyl with C₁-C₂₀, cycloalkyl with C₁-C₂₀, alkenyl with C₁-C₂₀, alkinyl with C₁-C₂₀, aryl, heteroaryl, or
- optionally a and f or b and e are NR, with R = alkyl with C₁-C₂₀, cycloalkyl with C₁-C₂₀, alkenyl with C₁-C₂₀, alkinyl with C₁-C₂₀, aryl, heteroaryl,
- wherein the bonds a-c, b-d, c-e and d-f, but not a-c and c-e at the same time and not b-d and d-f at the same time, can be unsaturated,
- wherein the bonds a-c, b-d, c-e and d-f can be part of a saturated or unsaturated ring system which can also contain the heteroelements O, S, N, P, Se, Ge, Sn, or
- the bonds a-c, b-d, c-e and d-f are part of an aromatic or condensed aromatic ring system which can also contain the heteroelements O, S, Si, N,
- wherein the atom E is a main group element,
- wherein the structure element a-E-b optionally is a constituent of a saturated or unsaturated ring system which can also contain the heteroelements O, S, Se, N, P, Si, Ge, Sn, or
- the structure element a-E-b optionally is a constituent of an aromatic ring system which can also contain the heteroelements O, S, Se, N.

11. Semiconductor material according to Claim 10, **characterised in that** M is Mo or W.

12. Semiconductor material according to Claim 10, **characterised in that** R₉, R₁₁, R₁₃, R₁₅, R₁₇, R₁₉ = H and R₁₀, R₁₂, R₁₄, R₁₆, R₁₈, R₂₀ = alkyl with C₁-C₂₀, cycloalkyl with C₁-C₂₀, alkenyl with C₁-C₂₀, alkinyl with C₁-C₂₀, aryl, heteroaryl, -NRR or -OR.

13. Semiconductor material according to Claim 10, **characterised in that** E is selected from the group of C, N, P, As, Sb.

14. Semiconductor material according to Claim 10, **characterised in that** the dopant has the following structure Ia:

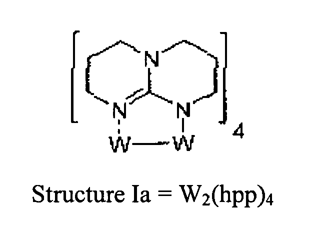

15. Semiconductor material according to one of Claims 6 to 8, **characterised in that** the dopant is an alkaline and/or alkaline earth metal.

16. Semiconductor material according to Claim 15, **characterised in that** the dopant is cesium.

17. Semiconductor material according to one of the preceding claims, **characterised in that** the matrix material has an energy level for the lowest unoccupied molecule orbital (LUMO) which differs by 0 - 0.5 V from the ionisation potential (HOMO) of the dopant.

18. Semiconductor material according to Claim 17, **characterised in that** the energy level of the matrix material differs by 0 - 0.3 V from the ionisation potential of the dopant.

19. Semiconductor material according to Claim 17, **characterised in that** the energy level of the matrix material differs by 0 - 0.15 V from the ionisation potential of the dopant.

20. Semiconductor material according to one of Claims 6 to 19, **characterised in that** the matrix material has a LUMO energy level which is lower than the ionisation potential (HOMO) of the dopant.

21. Semiconductor material according to one of the preceding Claims 6 to 20, **characterised in that** the concentration of the dopant is 0.5 to 25 weight percent.

22. Semiconductor material according to Claim 21, **characterised in that** the concentration of the dopant is 1 to 10 weight percent.

23. Semiconductor material according to Claim 21, **characterised in that** the concentration of the dopant is 2.5 to 5 weight percent.

24. Semiconductor material according to one of the preceding Claims 6 to 23, **characterised in that** the glass transition temperature Tg of the matrix material is higher than that of 4,7-diphenyl-1,10-phenanthroline.

25. Semiconductor material according to one of the preceding Claims 6 to 24, **characterised in that** the matrix material has an evaporation temperature of at least 200°C.

26. Organic light-emitting diode, comprising a semiconductor material according to any one of the preceding Claims 6 to 25.

27. Benzylidene hydroquinolinone of the formula 5 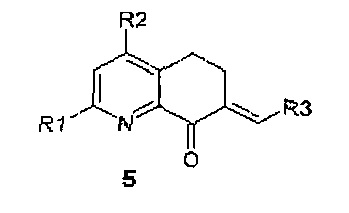 wherein R¹, R² and R³ are defined as in Claim 1.

## Revendications

1. Pyrido[3,2-h]chinazoline de la structure 8 suivante : 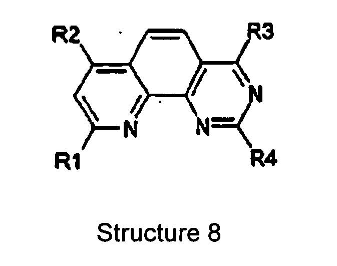 et/ou leurs 5,6-dihydrodérivés
où :
R¹ et R², substitué ou non substitué, est: aryle, hétéroaryle, alkyle de formule CHR₂ avec R = alkyle avec C₁-C₂₀ ou alkyle de formule CR₃ avec R = alkyle avec C₁-C₂₀;
R³ est sélectionné parmi H, substitué ou non substitué, alkyle avec C₁-C₂₀, aryle et hétéroaryle;
R⁴ est sélectionné parmi H, substitué ou non substitué, alkyle avec C₁-C₂₀, aryle, hétéroaryle, NH₂, NHR avec R = alkyle avec C₁-C₂₀, NR₂ avec R = alkyle avec C₁-C₂₀, N-alkylaryle, N-aryle₂, carbazolyle, dibenzazépinyle et CN.

2. Procédé de fabrication de pyrido[3,2-h]chinazolinène et/ou leurs 5,6-dihydrodérivés qui comprend les étapes suivantes :
(i) Transformation d'une 2,4-quinolinone disubstituée de structure 4 avec un aldéhyde en présence d'une base en vue de la représentation d'une benzylidène-dihydroquinolinone 5 conformément au schéma de réaction suivant : 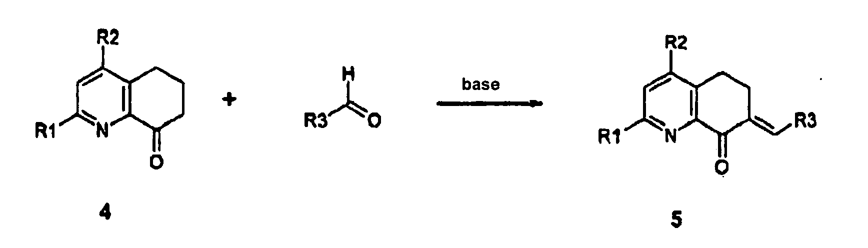
(ii) Transformation de la benzylidène-dihydroquinolinone 5 avec de l'hydrochlorure de benzamidinium dans des conditions basiques en vue de la représentation d'une 1,4,5,6-tétrahydro-pyrido[3,2-h]quinazoline 6, suivie de l'oxydation en vue de la représentation d'une 5,6-dihydropyrido[3,2-h]quinazoline 7 conformément au schéma de réaction suivant : 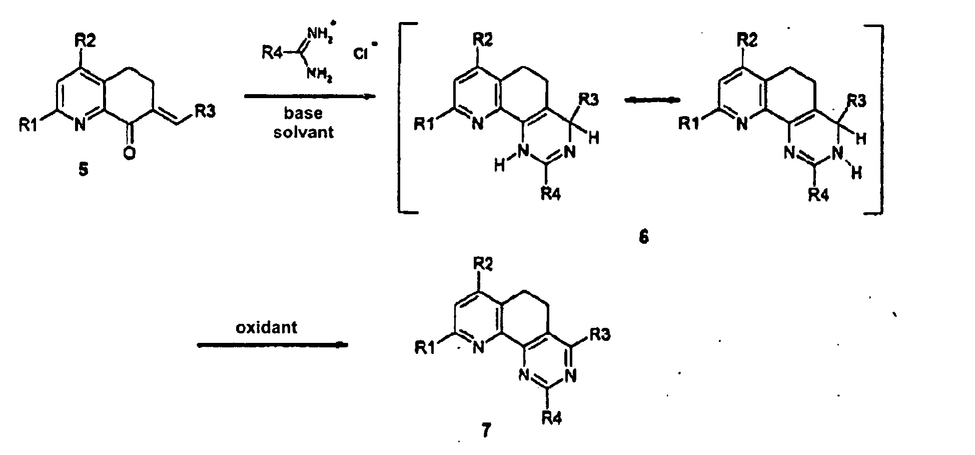
(iii) en option, aromatisation de la 5,6-dihydro-pyrido[3,2-h]quinazoline 7 en pyrido[3,2-h]quinazoline 8 conformément au schéma de réaction suivant : 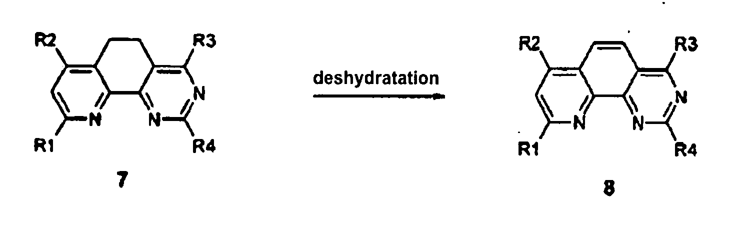 où R¹, R², R³ et R⁴ sont définis comme ci-dessus à la revendication 1.

3. Procédé selon la revendication 2, **caractérisé en ce qu'**on utilise comme base de l'hydroxyde de potassium et/ou du tert-butylate de potassium.

4. Procédé selon la revendication 2 ou 3, **caractérisé en ce que** l'on utilise du chloranile pour l'oxydation de la 1,4,5,6-tétrahydro-pyrido[3,2-h]quinazoline 6.

5. Procédé selon l'une quelconque des revendications 2 à 4, **caractérisé en ce que** l'aromatisation se fait par déshydratation catalysée par Pd au moyen de Pd/C.

6. Matériau semi-conducteur organique dopé, comprenant au moins un matériau de matrice organique qui est dopé avec au moins un dopant, le matériau de matrice étant une pyrido[3,2-h]quinazoline et/ou un 5,6-dihydrodérivé selon la revendication 1

7. Matériau semi-conducteur selon la revendication 6, **caractérisé en ce que** le matériau de matrice est réductible de manière réversible.

8. Matériau semi-conducteur selon la revendication 6, **caractérisé en ce que** le matériau de matrice se décompose lors d'une réduction en composants réactifs redox stables.

9. Matériau semi-conducteur selon l'une quelconque des revendications 6 à 8, **caractérisé en ce que** le dopant est un complexe métallique.

10. Matériau semi-conducteur selon la revendication 9, **caractérisé en ce que** le complexe métallique présente une structure I : 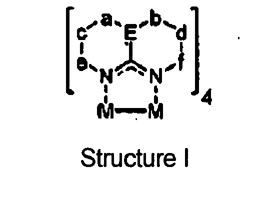 où M est un métal transitoire ; et où
- les éléments structurels a-f peuvent avoir la signification suivante : a = - CR₉R₁₀-, b = -CR₁₁R₁₂-, c = -CR₁₃R₁₄-, d = -CR₁₅R₁₆-, e = -CR₁₇R₁₈- et f = - CR₁₉R₂₀-, où R₉-R₂₀ sont indépendamment l'hydrogène, l'alkyle avec C₁-C₂₀, le cycloalkyle avec C₁-C₂₀, l'alkényle avec C₁-C₂₀, l'alkinyle avec C₁-C₂₀, l'aryle, l'hétéroaryle, -NRR ou -OR, où R = alkyle avec C₁-C₂₀, cycloalkyle avec C₁-C₂₀, alkényle avec C₁-C₂₀, alkinyle avec C₁-C₂₀, aryle ou hétéroaryle, ou
- chez les éléments structurels c et/ou d, C peut être remplacé par Si, ou
- au choix a ou b ou e ou f est NR, avec R = alkyle avec C₁-C₂₀, cycloalkyle avec C₁-C₂₀, alkényle avec C₁-C₂₀, alkinyle avec C₁-C₂₀, aryle, hétéroaryle, ou
- au choix a et f ou b et e sont NR, avec R = alkyle avec C₁-C₂₀, cycloalkyle avec C₁-C₂₀, alkényle avec C₁-C₂₀, alkinyle avec C₁-C₂₀, aryle, hétéroaryle,
- les liaisons a-c, b-d, c-e et d-f, mais pas simultanément a-c et c-e et pas simultanément b-d et d-f pouvant être non saturées,
- les liaisons a-c, b-d, c-e et d-f pouvant faire partie d'un système annulaire saturé ou non saturé qui peut également contenir les hétéroéléments O, S, Se, N, P, Ge, Sn, ou
- les liaisons a-c, b-d, c-e et d-f font partie d'un système annulaire aromatique ou aromatique condensé qui peut également contenir les hétéroéléments O, S, Si, N,
- l'atome E étant un élément du groupe principal,
- l'élément structurel a-E-b étant au choix partie intégrante d'un système annulaire saturé ou non saturé qui peut également contenir les hétéroéléments O, S, Se, N, P, Si, Ge, Sn, ou
- l'élément structurel a-E-b étant au choix partie intégrante d'un système annulaire aromatique qui peut également contenir les hétéroéléments O, S, Se, N.

11. Matériau semi-conducteur selon la revendication 10, **caractérisé en ce que** M est Mo ou W.

12. Matériau semi-conducteur selon la revendication 10, **caractérisé en ce que** R₉, R₁₁, R₁₃, R₁₅, R₁₇, R₁₉ = H et R₁₀, R₁₂, R₁₄, R₁₆, R₁₈, R₂₀ = alkyle avec C₁-C₂₀, cycloalkyle avec C₁-C₂₀, alkényle avec C₁-C₂₀, alkinyle avec C₁-C₂₀, aryle, hétéroaryle, -NRR ou -OR.

13. Matériau semi-conducteur selon la revendication 10, **caractérisé en ce que** l'atome E est sélectionné dans le groupe C, N, P, As, Sb.

14. Matériau semi-conducteur selon la revendication 10, **caractérisé en ce que le** dopant présente la structure la suivante :

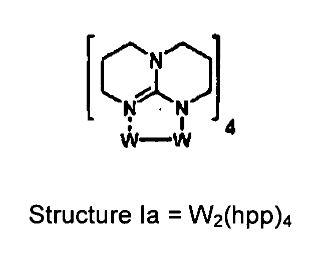

15. Matériau semi-conducteur selon l'une quelconque des revendications 6 à 8, **caractérisé en ce que** le dopant est un métal alcalin et/ou un métal alcalinoterreux.

16. Matériau semi-conducteur selon la revendication 15, **caractérisé en ce que** le dopant est le césium.

17. Matériau semi-conducteur selon l'une quelconque des revendications précédentes, **caractérisé en ce que** le matériau de matrice présente un niveau d'énergie pour la plus basse orbite moléculaire non occupée (LUMO) qui diffère de 0 - 0,5 V du potentiel d'ionisation (HOMO) du dopant.

18. Matériau semi-conducteur selon la revendication 17, **caractérisé en ce que** le niveau d'énergie du matériau de matrice diffère de 0 - 0,3 V du potentiel d'ionisation du dopant.

19. Matériau semi-conducteur selon la revendication 17, **caractérisé en ce que** le niveau d'énergie du matériau de matrice diffère de 0 - 0,15 V du potentiel d'ionisation du dopant.

20. Matériau semi-conducteur selon l'une quelconque des revendications 6 à 19, **caractérisé en ce que** le matériau de matrice présente un niveau d'énergie LUMO qui est inférieur au potentiel d'ionisation (HOMO) du dopant.

21. Matériau semi-conducteur selon l'une quelconque des revendications précédentes 6 à 20, **caractérisé en ce que** la concentration du dopant est égale à 0,5 à 25 pourcent en masse.

22. Matériau semi-conducteur selon la revendication 21, **caractérisé en ce que** la concentration du dopant est égale à 1 à 10 pourcent en masse.

23. Matériau semi-conducteur selon la revendication 21, **caractérisé en ce que** la concentration du dopant est égale à 2,5 à 5 pourcent en masse.

24. Matériau semi-conducteur selon l'une quelconque des revendications précédentes 6 à 23, **caractérisé en ce que** la température de transition de verre Tg du matériau de matrice est supérieure à celle de la 4,7-diphényle-1,10-phénantroline.

25. Matériau semi-conducteur selon l'une quelconque des revendications précédentes 6 à 24, **caractérisé en ce que** le matériau de matrice présente une température d'évaporation au moins égale à 200°C.

26. Diode électroluminescente organique qui comprend un matériau semi-conducteur selon l'une quelconque des revendications précédentes 6 à 25.

27. Benzylidène-hydroquinolinone de la formule 5 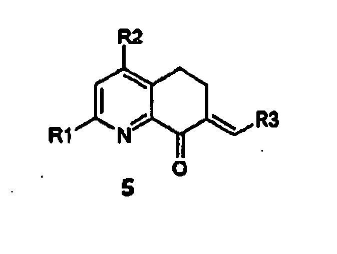 où R¹, R² et R³ sont définis comme à la revendication 1.
